# EUROPEAN PATENT SPECIFICATION

(11) **EP 3 240 612 B1**
(45) Date of publication and mention of the grant of the patent: **23.12.2020**
(21) Application number: 15875361.6
(22) Date of filing: 30.04.2015
(51) Int. Cl.: C12N 5/079, A61K 35/36

(54) **METHODS OF TREATING RETINAL DISEASES**
VERFAHREN ZUR BEHANDLUNG VON NETZHAUTERKRANKUNGEN
PROCÉDÉS DE TRAITEMENT DE MALADIES RÉTINIENNES

(30) Priority: 30.12.2014 US 201462097753 P; 17.02.2015 US 201562116980 P; 17.02.2015 US 201562116972 P
(43) Date of publication of application: 08.11.2017
(73) Proprietor: Cell Cure Neurosciences Ltd., 9112102 Jerusalem (IL)
(72) Inventor: BANIN, Eyal, 96954 Jerusalem (IL); REUBINOFF, Benjamin Eithan, 9988000 Doar-Na HaEla (IL); BOHANA-KASHTAN, Osnat, 4060000 Tel-Mond (IL); NETZER, Nir, 76804 Mazkeret Batia (IL); IRVING, Charles Sherard, 3088900 Caesarea (IL)
(74) Representative: Hoffmann Eitle
(86) International application number: PCT/IL2015/050456
(87) International publication number: WO 2016/108219

(56) References cited:
- WO-A1-2013/074681
- WO-A1-2015/087231
- US-A1- 2013 196 369
- HANNA VAAJASAARI ET AL: "Toward the defined and xeno-free differentiation of functional human pluripotent stem cell-derived retinal pigment epithelial cells.", MOLECULAR VISION, vol. 17, 1 January 2011 (2011-01-01), pages 558-575, XP055108414, ISSN: 1090-0535
- BUZHOR ELLA ET AL: "Cell-based therapy approaches: the hope for incurable diseases", REGENERATIVE MEDICINE, FUTURE MEDICINE, UK, vol. 9, no. 5, 1 January 2014 (2014-01-01) , pages 649-672, XP009188959, ISSN: 1746-076X, DOI: 10.2217/RME.14.35
- SHELLY E. TANNENBAUM ET AL: "Derivation of Xeno-Free and GMP-Grade Human Embryonic Stem Cells - Platforms for Future Clinical Applications", PLOS ONE, vol. 7, no. 6, 20 June 2012 (2012-06-20), page e35325, XP055471292, DOI: 10.1371/journal.pone.0035325
- VUGLER, ANTHONY ET AL.: 'Elucidating the phenomenon of HESC-derived RPE: anatomy of cell genesis, expansion and retinal transplantation.' EXPERIMENTAL NEUROLOGY vol. 214, no. 2, 2008, pages 347 - 361, XP025695837 Retrieved from the Internet: <URL:http://www. researchgate.net/publication/23389123_Eluci dating_the_phenomenon_of_HESC- derived_RPE_anatomy_of_ cell _genesis_expansion_and_retinal_transplantat ion._Exp_Neurol> [retrieved on 2015-07-06]
- KAMAO, HIROYUKI ET AL.: 'Characterization of human induced pluripotent stem cell -derived retinal pigment epithelium cell sheets aiming for clinical application.' STEM CELL REPORTS vol. 2, no. 2, 2014, pages 205 - 218, XP055144196 Retrieved from the Internet: <URL:http://www.sciencedirect.com/science/a rticle/pii/52213671113001756> [retrieved on 2015-07-06]
- LUND, RAYMOND D. ET AL.: 'Human embryonic stem cell -derived cells rescue visual function in dystrophic RCS rats.' CLONING AND STEM CELLS vol. 8, no. 3, 29 September 2006, pages 189 - 199, XP002528361 Retrieved from the Internet: <URL:http://online.liebertpub.com/doi/abs/1 0.1089/clo.2006.8.189> [retrieved on 2015-07-06]
- IDELSON, MARIA ET AL.: 'Directed differentiation of human embryonic stem cells into functional retinal pigment epithelium cells.' CELL STEM CELL vol. 5, no. 4, pages 396 - 408, XP055046648 Retrieved from the Internet: <URL:http://www.sciencedirect.com/science/a rticle/pii/S1934590909003361> [retrieved on 2015-07-06]
- CLEGG, DENNIS O. ET AL.: 'Derivation of retinal pigmented epithelial cells for the treatment of ocular disease.' STEM CELLS HANDBOOK. 03 July 2013, NEW YORK, pages 411 - 418, XP055392476 Retrieved from the Internet: <URL:http://link.springer.com/chapter/10.10 07/978-1-4614-7696-2_29> [retrieved on 2015-07-06]

## Description

### FIELD AND BACKGROUND OF THE INVENTION

The present invention, in some embodiments thereof, relates to methods of treating retinal diseases and more particularly age-related macular degeneration (AMD).

The derivation of hESCs more than a decade ago has raised immense interest in the potential clinical use of these cells for regeneration by serving as the starting material for therapeutic cells. While hESC-derived cells have not yet been approved for clinical use, significant progress has been made through the years towards fulfilling this goal. Important advancements include better understanding of the biology of hESCs, technological improvements in the ability to differentiate them to various cell types and preclinical proof of their therapeutic effect in disease-specific animal models.

The RPE is a monolayer of pigmented cells which lies between the neural retina and the choriocapillaris. The RPE is characterized by an apical to basolateral structural and functional polarity. On the apical side, the cells make direct contact with the photoreceptors. On their lateral walls they form tight, adherent gap junctions and on their basal side, they contact the underlying Bruch's basal membrane which separates them from the choroidal blood vessels. The RPE cells play crucial roles in the maintenance and function of the retina and its photoreceptors. These include the formation of the blood-retinal barrier, absorption of stray light, supply of nutrients to the neural retina, regeneration of visual pigment and uptake and recycling of shed outer segments of photoreceptors.

Dysfunction, degeneration and loss of RPE cells are prominent features of AMD, Best Disease and subtypes of Retinitis Pigmentosa (RP). AMD is the leading cause of visual disability in the Western world. Among people over 75 years of age, 25-30% are affected by Age-Related Macular Degeneration (AMD), with progressive central visual loss that leads to blindness in 6-8% of the patients. The retinal degeneration primarily involves the macula, the central part of the retina responsible for fine visual detail and color perception. The dry form of AMD is initiated by hyperplasia of the RPE and formation of drusen deposits underneath the RPE or within the Bruch's membrane consisting of metabolic end products. It may gradually progress into the advanced stage of geographic atrophy (GA) with degeneration of RPE cells and photoreceptors over large areas of the macula, causing central visual loss. Ten percent of dry AMD patients will progress to neovascular (wet) AMD, with blood vessels sprouting through the Bruch's membrane and with subsequent intraocular leakage and/or bleeding, accelerating the loss of central vision. While the complicating neovascularization can be treated with anti-VEGF agents, currently there is no effective treatment to halt RPE and photoreceptor degeneration and many patients will eventually lose their sight.

Transplantation studies both in animals and in humans provide evidence for the potential therapeutic effect of transplanting RPE cells in AMD patients. In humans, macular translocation onto more peripheral RPE, as well as autologous transplantation of peripheral RPE as cell suspensions or patches of RPE and choroid, provide proof-of-principle that positioning the macula above relatively more healthy RPE cells can improve visual function in some AMD patients. Nevertheless, the surgical procedures for autologous grafting are challenging and are associated with significant complications. Typically, RPE cells are delivered through a small retinotomy following a standard 3-port vitrectomy to a subretinal space created in the macular area along the border between areas of GA and better preserved extra-foveal retinal and RPE layer. Success of such cellular replacement strategy in treating AMD is contingent on establishing a safe delivery system that enables survival and function of the transplanted cells and minimizes retinal damage.

Background art includes WO 2013/114360, WO 2013/074681, WO 2008/129554 and WO 2013/184809, US Patent Application No. 62/116,972 and US Patent Application No. 62/116,980.

### SUMMARY OF THE INVENTION

According to an aspect of some embodiments of the present invention there is provided a method of treating a subject with dry-form age-related macular degeneration (AMD) comprising administering into the subretina of the subject a therapeutically effective amount of a pharmaceutical composition comprising human RPE cells, wherein at least 95 % of the cells thereof co-express premelanosome protein (PMEL17) and cellular retinaldehyde binding protein (CRALBP), wherein the trans-epithelial electrical resistance of the cells in a monolayer is greater than 100 ohms to the subject, thereby treating the subject.

According to an aspect of some embodiments of the present invention there is provided a method of treating a subject with a retinal disease or condition comprising administering into the retina of the subject a therapeutically effective amount of a pharmaceutical composition comprising human polygonal RPE cells, wherein at least 95 % of the cells thereof co-express premelanosome protein (PMEL17) and cellular retinaldehyde binding protein (CRALBP), wherein the trans-epithelial electrical resistance of the cells in a monolayer is greater than 100 ohms to the subject, wherein the therapeutically effective amount is between 50,000 - 5,000,000 cells per administration, thereby treating the subject.

According to an aspect of some embodiments of the present invention there is provided a method of treating a subject with a retinal disease or condition comprising administering into the retina of the subject a therapeutically effective amount of a pharmaceutical composition comprising human RPE cells using a device, wherein the outer diameter of the device through which the cells are administered is between 90-100 µm, thereby treating the subject, and wherein at least 95 % of the cells thereof co-express premelanosome protein (PMEL17) and cellular retinaldehyde binding protein (CRALBP), wherein the trans-epithelial electrical resistance of the cells in a monolayer is greater than 100 ohms to the subject.

According to some embodiments of the invention, the device is a cannula.

According to some embodiments of the invention, the therapeutically effective amount is between 50,000-1,000,000 cells per administration.

According to some embodiments of the invention, the therapeutically effective amount is selected from the group consisting of 50,000 cells per administration, 200,000 cells per administration, 500,000 cells per administration and 1,000,000 cells per administration.

According to some embodiments of the invention, the cells are administered into the subretinal space of the subject.

According to some embodiments of the invention, the cells are administered in a single administration.

According to some embodiments of the invention, the pharmaceutical composition comprises 500 cells per µl - 10,000 cells per µl.

According to some embodiments of the invention, when the amount is 50,000 cells per administration, the pharmaceutical composition comprises about 500-1000 cells per µl.

According to some embodiments of the invention, when the amount is 200,000 cells per administration, the pharmaceutical composition comprises about 2,000 cells per µl.

According to some embodiments of the invention, when the amount is 500,000 cells per administration, the pharmaceutical composition comprises about 5,000 cells per µl.

According to some embodiments of the invention, when the amount is 1,000,000 cells per administration, the pharmaceutical composition comprises about 10,000 cells per µl.

According to some embodiments of the invention, the retinal disease or condition is selected from the group consisting of retinitis pigmentosa, retinal detachment, retinal dysplasia, retinal atrophy, retinopathy, macular dystrophy, cone dystrophy, cone-rod dystrophy, Malattia Leventinese, Doyne honeycomb dystrophy, Sorsby's dystrophy, pattern/butterfly dystrophies, Best vitelliform dystrophy, North Carolina dystrophy, central areolar choroidal dystrophy, angioid streaks, toxic maculopathy, Stargardt disease, pathologic myopia, retinitis pigmentosa, and macular degeneration.

According to some embodiments of the invention, the disease is age-related macular degeneration.

According to some embodiments of the invention, the age-related macular degeneration is dry-form age-related macular degeneration.

According to some embodiments of the invention, the subject fulfils at least one of the criteria selected from the group consisting of:
(i) is aged 55 or older;
(ii) has funduscopic findings of dry AMD with geographic atrophy in the macula, above 0.5 disc area in at least one eye;
(iii) is able to undergo a vitreoretinal surgical procedure under monitored anesthesia care; and
(iv) does not have an immunodeficiency disease.

According to some embodiments of the invention, the subject does not have a retinal disease other than AMD;

According to some embodiments of the invention, the subject fulfils each of the criteria (i) - (iv).

According to some embodiments of the invention, the outer aperture of the device through which the cells are administered is between 90-100 µm.

According to some embodiments of the invention, the device is a cannula.

According to some embodiments of the invention, the device further comprises a needle.

According to some embodiments of the invention, the gauge of the cannula is 25G.

According to some embodiments of the invention, the number of Oct4⁺TRA-1-60⁺ cells in the population is below 1:250,000.

According to some embodiments of the invention, at least 80 % of the cells express Bestrophin 1, as measured by immunostaining.

According to some embodiments of the invention, at least 80 % of the cells express Microphthalmia-associated transcription factor (MITF), as measured by immunostaining.

According to some embodiments of the invention, more than 80 % of the cells express paired box gene 6 (PAX-6) as measured by FACS.

According to some embodiments of the invention, the cells secrete greater than 500 ng of Pigment epithelium-derived factor (PEDF) per ml per day.

According to some embodiments of the invention, the cells secrete PEDF and vascular endothelial growth factor (VEGF) in a polarized manner.

According to some embodiments of the invention, the ratio of apical secretion of PEDF: basal secretion of PEDF is greater than 1.

According to some embodiments of the invention, the ratio remains greater than 1 following incubation for 8 hours at 2-8 ° C.

According to some embodiments of the invention, the trans-epithelial electrical resistance of the cells remains greater than 100 ohms following incubation for 8 hours at 2-8 ° C.

According to some embodiments of the invention, the ratio of basal secretion of VEGF: apical secretion of VEGF is greater than 1.

According to some embodiments of the invention, the ratio remains greater than 1 following incubation for 8 hours at 2-8 ° C.

According to some embodiments of the invention, the cells are capable of rescuing visual acuity in the RCS rat following subretinal administration.

According to some embodiments of the invention, the cells are capable of rescuing photoreceptors for up to 180 days post-subretinal administration in the RCS rat.

According to some embodiments of the invention, the cells are generated by ex-vivo differentiation of human embryonic stem cells.

According to some embodiments of the invention, the cells are generated by:
(a) culturing human embryonic stem cells or induced pluripotent stem cells in a medium comprising nicotinamide so as to generate differentiating cells, wherein the medium is devoid of activin A;
(b) culturing the differentiating cells in a medium comprising nicotinamide and acitivin A to generate cells which are further differentiated towards the RPE lineage; and
(c) culturing the cells which are further differentiated towards the RPE lineage in a medium comprising nicotinamide, wherein the medium is devoid of activin A.

According to some embodiments of the invention, the embryonic stem cells or induced pluripotent stem cells are propagated in a medium comprising bFGF and TGFβ.

According to some embodiments of the invention, the embryonic stem cells are cultured on human umbilical cord fibroblasts.

According to some embodiments of the invention, steps (a)-(c) are effected under conditions wherein the atmospheric oxygen level is less than about 10 %.

According to some embodiments of the invention, the method further comprises culturing embryonic stem cells or induced pluripotent stem cells in a medium under conditions wherein the atmospheric oxygen level is greater than about 10 % in the presence of nicotinamide prior to step (a).

According to some embodiments of the invention, the method further comprises culturing the differentiated cells in a medium under conditions wherein the atmospheric oxygen level is greater than about 10 % in the presence of nicotinamide following step (c).

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the description are herein described, by way of example only, with reference to the accompanying drawings. With specific reference now to the drawings in detail, it is stressed that the particulars shown are by way of example and for purposes of illustrative discussion of embodiments of the description. In this regard, the description taken with the drawings makes apparent to those skilled in the art how embodiments of the description may be practiced.

In the drawings:

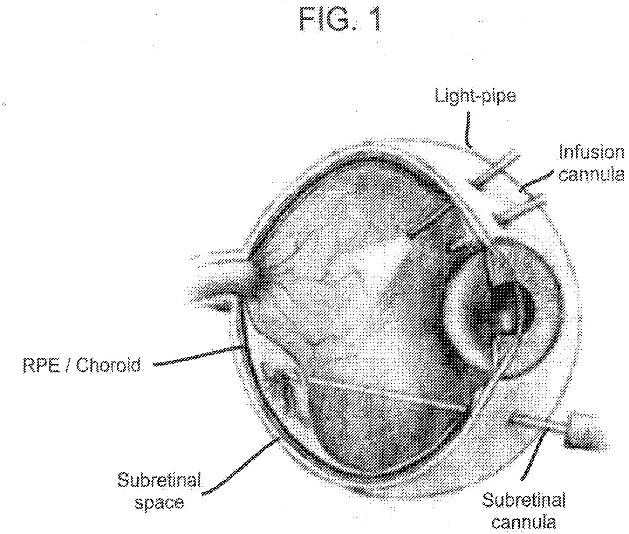

FIG. 1 is a drawing of the subretinal injection scheme. Note that in this drawing, the placement of the surgical ports is not anatomically accurate (Stout and Francis, 2011, Human Gene Ther. 2011 May 22(5): 531-5).
FIGs. 2A-C are a photograph illustrating assembly of the device of delivery and loading of the formulated cells. A. Syringe connected to 18G blunt fill needle. B. Replacement of the 18G blunt fill needle with the extension tube. C. The assembled device of delivery.

### DESCRIPTION OF SPECIFIC EMBODIMENTS OF THE INVENTION

The present invention, in some embodiments thereof, relates to methods of treating retinal diseases and more particularly age-related macular degeneration (AMD).

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

AMD is a progressive chronic disease of the central retina and a leading cause of vision loss worldwide. Most visual loss occurs in the late stages of the disease due to one of two processes: neovascular ("wet") AMD and geographic atrophy (GA, "dry"). In neovascular age-related macular degeneration, choroidal neovascularisation breaks through to the sub-RPE or subretinal space, leaking fluid, lipid, and blood and leading to fibrous scarring. In GA, progressive atrophy of the retinal pigment epithelium, choriocapillaris, and photoreceptors occurs. The dry form of AMD is more common (85-90% of all cases), but may progress to the "wet" form, which, if left untreated, leads to rapid and severe vision loss.

The estimated prevalence of AMD is 1 in 2,000 people in the US and other developed countries. This prevalence is expected to increase together with the proportion of elderly in the general population. The risk factors for the disease include both environmental and genetic factors.

The pathogenesis of the disease involves abnormalities in four functionally interrelated tissues, i.e., retinal pigment epithelium (RPE), Bruch's membrane, choriocapillaries and photoreceptors. However, impairment of RPE cell function is an early and crucial event in the molecular pathways leading to clinically relevant AMD changes.

There is currently no effective or approved treatment for dry-AMD. Prophylactic measures include vitamin/mineral supplements. These reduce the risk of developing wet AMD but do not affect the development of progression of geographic atrophy.

Currently, there are about twenty therapies in various stages of clinical development. Among these are complement system inhibitors and corticosteroids, visual cycle modulators, anti-oxidants, neuroprotectants, vascular enhancers and cell and gene therapies - see for example Dugel et al., 2014, Retina Today, pages 70-72; and Patel et al, 2015, Practical Retina, January 2015 · Vol. 46, No. 1, pages 8-13.

Human embryonic stem cells have been proposed as a cellular source for the generation of RPE cells. Two general approaches have been used to obtain RPE cells from hESCs, spontaneous differentiation and directed differentiation. In spontaneous differentiation, hESCs in flat colonies or in embryoid bodies (EBs) are allowed to spontaneously differentiate into a population of cells containing pigmented RPE cells. The directed differentiation method uses a number of factors to drive the differentiation of hESCs to RPE cells see for example WO 2008/129554.

The present inventors have now discovered optimal conditions for clinical treatment with PRE cells including doses and treatment regimens and devices for delivering the cells. The present invention further provides criteria for selection of populations of patients who would gain benefit by treatment with the cells.

Thus, according to one aspect of the present invention there is provided a method of treating a subject with a retinal disease (e.g. dry-form age-related macular degeneration (AMD)) comprising administering into the subretina of the subject a therapeutically effective amount of a pharmaceutical composition comprising human RPE cells, wherein at least 95 % of the cells thereof co-express premelanosome protein (PMEL17) and cellular retinaldehyde binding protein (CRALBP), wherein the trans-epithelial electrical resistance of the cells is greater than 100 ohms to the subject, thereby treating the subject.

Eye conditions for which the pharmaceutical compositions serve as therapeutics include, but are not limited to retinal diseases or disorders generally associated with retinal dysfunction, retinal injury, and/or loss of retinal pigment epithelium. A non-limiting list of conditions which may be treated in accordance with the invention comprises retinitis pigmentosa, lebers congenital amaurosis, hereditary or acquired macular degeneration, age related macular degeneration (AMD), Best disease, retinal detachment, gyrate atrophy, choroideremia, pattern dystrophy as well as other dystrophies of the RPE, Stargardt disease, RPE and retinal damage due to damage caused by any one of photic, laser, inflammatory, infectious, radiation, neo vascular or traumatic injury.

According to a particular embodiment, the disease is dry-form age-related macular degeneration.

Subjects which may be treated include primate (including humans), canine, feline, ungulate (e.g., equine, bovine, swine (e.g., pig)), avian, and other subjects. Humans and non- human animals having commercial importance (e.g., livestock and domesticated animals) are of particular interest. Exemplary mammals which may be treated include, canines; felines; equines; bovines; ovines; rodentia, etc. and primates, particularly humans. Non-human animal models, particularly mammals, e.g. primate, murine, lagomorpha, etc. may be used for experimental investigations.

According to one embodiment, the subject who is treated is aged 55 or older.

According to another embodiment, the subject who is treated has funduscopic findings of dry AMD with geographic atrophy in the macula, above 0.5 disc area (1.25mm² and up to 17 mm²) in at least one eye.

According to still another embodiment, the subject who is treated is in a condition such that he is able to undergo a vitreoretinal surgical procedure under monitored anesthesia care;

According to still another embodiment, the subject does not have an immunodeficiency disease.

According to still another embodiment, the subject does not have axial myopia greater than -6 diopters.

According to still another embodiment, the subject does not have a history of retinal detachment repair.

According to still another embodiment, the subject does not have a retinal disease other than AMD.

Preferably, the subject who is treated fulfils at least one, two, three, four, five, six or all of the above criteria.

The RPE cells generated as described herein may be transplanted to various target sites within a subject's eye. In accordance with one embodiment, the transplantation of the RPE cells is to the subretinal space of the eye, which is the normal anatomical location of the RPE (between the photoreceptor outer segments and the choroids). In addition, dependent upon migratory ability and/or positive paracrine effects of the cells, transplantation into additional ocular compartments can be considered including the vitreal space, inner or outer retina, the retinal periphery and within the choroids.

The number of viable cells that may be administered to the subject are typically between 50,000-5x10⁶, between 50,000-4x10⁶ between 50,000-3x10⁶ between 50,000-2x10⁶ between 50,000-1x10⁶, between 50,000-500,000 per injection.

The present invention contemplates a single administration or multiple administrations. Preferably, the time between a first administration to an eye and a second administration to the same eye is at least one month.

According to a specific embodiment, the number of viable cells that may be administered per eye of the subject are between 50,000-2x10⁶, between 50,000-1x10⁶, between 50,000-500,000. Exemplary doses include 50,000 cells per eye, 100,000 cells per eye, 200,000 cells per eye, 300,000 cells per eye, 400,000 cells per eye, 500,000 cells per eye and 1x10⁶ cells per eye.

The cells are typically formulated in a carrier (e.g. an isotonic solution and/or a saline) such as BSS plus™. The carrier may optionally comprise additional factors that support RPE engraftment, integration, survival, potency etc.

An exemplary concentration of a pharmaceutical composition comprising 50,000 cells is about 500 cells per µl or 1,000 cells per µl. An exemplary concentration of a pharmaceutical composition comprising 200,000 viable cells is about 500 viable cells per µl or 1,000 viable cells per µl. An exemplary concentration of a pharmaceutical composition comprising 500,000 cells is about 5,000 viable cells per µl or about 10,000 viable cells per µl. An exemplary concentration of a pharmaceutical composition comprising 1x10⁶cells is about 10,000 viable cells per µl.

The transplantation may be performed by various techniques known in the art. Methods for performing RPE transplants are described in, for example, U.S. Pat. Nos. 5,962,027, 6,045,791, and 5,941,250 and in Eye Graefes Arch Clin Exp Opthalmol March 1997; 235(3):149-58; Biochem Biophys Res Commun Feb. 24, 2000; 268(3): 842-6; Opthalmic Surg February 1991; 22(2): 102-8. Methods for performing corneal transplants are described in, for example, U.S. Pat. No. 5,755,785, and in Eye 1995; 9 (Pt 6 Su):6-12; Curr Opin Opthalmol August 1992; 3 (4): 473-81; Ophthalmic Surg Lasers April 1998; 29 (4): 305-8; Ophthalmology April 2000; 107 (4): 719-24; and Jpn J Ophthalmol November-December 1999; 43(6): 502-8. If mainly paracrine effects are to be utilized, cells may also be delivered and maintained in the eye encapsulated within a semi-permeable container, which will also decrease exposure of the cells to the host immune system (Neurotech USA CNTF delivery system; PNAS March 7, 2006 vol. 103(10)3896-3901).

The step of administering may comprise intraocular administration of the RPE cells into an eye in need thereof. The intraocular administration may comprise injection of the RPE cells into the subretinal space.

In accordance with one embodiment, transplantation is performed via pars plana vitrectomy surgery followed by delivery of the cells through a small retinal opening into the sub-retinal space or by direct injection.

The RPE cells may be transplanted in various forms. For example, the RPE cells may be introduced into the target site in the form of cell suspension, with matrix or adhered onto a matrix or a membrane, extracellular matrix or substrate such as a biodegradable polymer or a combination. The RPE cells may also be transplanted together (co-transplantation) with other retinal cells, such as with photoreceptors.

According to a particular embodiment, an aqueous solution (e.g. an isotonic solution and/or a saline) or air is administered into the subretinal space, thereby forming an initial bleb. Then the RPE cells as a suspension or upon a scaffold are administered into the same subretinal space. The injection may be through a needle or injection cannula.

According to a particular embodiment, the cells are delivered as a cell suspension using a delivery device (e.g. needle or injection cannula) which has an outer diameter between 90-100 µm. According to another embodiment, the cells are delivered as a cell suspension using a delivery device (e.g. needle or injection cannula) which has an inner aperture diameter between 65-75 µm.

According to embodiments of this aspect of the present invention the RPE cells in their final formulation at a concentration of 70,000-1.4 x10⁶ viable cells/100 µl are loaded into a delivery device (e.g. 1 mL syringe) using a 18G needle (70,000 cells are loaded for the 50,000 dose, 700,000 cells are loaded for the 500,000 dose and 1.4 x10⁶ cells are loaded for the 1 x10⁶ dose). The 18G needle may then be replaced with an extension tube (e.g. between 5-10 cm) and air is removed through the extension tube. An injection cannula which has a tip having an outer diameter between 90-100 µm (e.g. 41G) may then be attached to the end of the extension tube. According to another embodiment, the inner diameter of the aperture of the tip is about 65-75 µm (e.g. about 70 µm).

The concentration of the RPE cells upon loading into the needle or injection cannula may be between about 2,000 viable cells/µl and about 14,000 viable cells/µl. The concentration of viable RPE cells to be delivered from the needle or injection cannula may be between about 1,000 viable cells/µl and about 10,000 viable cells/µl.

According to a particular embodiment, the cannula comprises a 41G tip(example as manufactured by Peregrine). According to still another embodiment, the cannula is a 25G cannula.

The present invention provides for an article of manufacture comprising an 18G needle and a 25G/41G cannula. Such a device may be used for the uptake of RPE cells and the subsequent intraocular administration of RPE cells.

The device may further comprise an extension tube (e.g. between about 5 and 15 cm) and a syringe (e.g. 1-2 ml syringe).

According to another aspect there is provided an article of manufacture comprising a 25G/41G cannula, a syringe (e.g. 1-2 ml syringe) and an extension tube (e.g. between about 5 and 15 cm). The article of manufacture may further comprise an 18G needle.

The effectiveness of treatment may be assessed by different measures of visual and ocular function and structure, including, among others, best corrected visual acuity (BCVA), retinal sensitivity to light as measured by perimetry or microperimetry in the dark and light-adapted states, full-field, multi-focal, focal or pattern elecroretinography ERG), contrast sensitivity, reading speed, color vision, clinical biomicroscopic examination, fundus photography, optical coherence tomography (OCT), fundus autofluorescence (FAF), infrared and multicolor imaging, fluorescein or ICG angiography, and additional means used to evaluate visual function and ocular structure.

The subject may be administered corticosteroids prior to or concurrently with the administration of the RPE cells, such as prednisolone or methylprednisolone, Predforte.

According to another embodiment, the subject is not administered corticosteroids prior to or concurrently with the administration of the RPE cells, such as prednisolone or methylprednisolone, Predforte.

Immunosuppressive drugs may be administered to the subject prior to, concurrently with and/or following treatment.

The immunosuppressive drug may belong to the following classes:
Glucocorticoids, Cytostatics (e.g. alkylating agent or antimetabolite), antibodies (polyclonal or monoclonal), drugs acting on immunophilins (e.g. ciclosporin, Tacrolimus or Sirolimus). Additional drugs include interferons, opiods, TNF binding proteins, mycophenolate and small biological agents.

Examples of immunosuppressive drugs include: mesenchymal stem cells, antilymphocyte globulin (ALG) polyclonal antibody, anti-thymocyte globulin (ATG) polyclonal antibody, azathioprine, BAS1 L1X1MAB® (anti-I L-2Ra receptor antibody), cyclosporin (cyclosporin A), DACLIZUMAB® (anti-I L-2Ra receptor antibody), everolimus, mycophenolic acid, RITUX1MAB® (anti-CD20 antibody), sirolimus, tacrolimus, Tacrolimus and or Mycophenolate mofetil.

Antibiotics may be administered to the subject prior to, concurrently with and/or following treatment. Examples of antibiotics include Oflox, Gentamicin, Chloramphenicol, Tobrex, Vigamox or any other topical antibiotic preparation authorized for ocular use.

"Retinal pigment epithelium cells", "RPE cells", "RPEs", which may be used interchangeably as the context allows, refers to cells of a cell type functionally similar to that of native RPE cells which form the pigment epithelium cell layer of the retina (e.g. upon transplantation within an eye, they exhibit functional activities similar to those of native RPE cells). Thus, the terms "retinal pigment epithelium cells", "RPE cells", or "RPEs" may be used to refer to both native RPE cells of the pigmented layer of the retina and RPE cells directly differentiated from human stem cells (hSCs), in accordance with the present disclosure.

The term "hSC-derived RPE cells" is used herein to denote RPE cells that are obtained by directed differentiation from hSCs. In accordance with a preferred embodiment, the hSC-derived RPE cells are functional RPE cells as exhibited by parameters defined herein below. The term "directed differentiation" is used interchangeably with the term "RPE induced differentiation" and is to be understood as meaning the process of manipulating hSCs under culture conditions which induce/promote differentiation into the RPE cell type.

According to a particular embodiment, the RPE cells are obtained by directed differentiation of hSCs in the presence of one or more members of the TGFβ superfamily, and exhibit at least one of the following characteristics:
- during differentiation, the cultured cells respond to TGFβ signaling;
- the RPE cells express markers indicative of terminal differentiation, e.g. bestrophin 1, CRALBP and/or RPE65;
- following transplantation (i.e. in situ), the RPE cells exhibit trophic effect supporting photoreceptors adjacent to RPE cells;
- further, in situ the RPE cells are capable of functioning with phagocytosis of shed photoreceptor outer segments as part of the normal renewal process of these photoreceptors;
- further, in situ the RPE cells are capable of generating a retinal barrier and functioning in the visual cycle.

As used herein, the phrase "stem cells" refers to cells which are capable of remaining in an undifferentiated state (*e.g.,* pluripotent or multipotent stem cells) for extended periods of time in culture until induced to differentiate into other cell types having a particular, specialized function (*e.g.,* fully differentiated cells). Preferably, the phrase "stem cells" encompasses embryonic stem cells (ESCs), induced pluripotent stem cells (iPSCs), adult stem cells, mesenchymal stem cells and hematopoietic stem cells.

According to a particular embodiment, the RPE cells are generated from ESC.

The phrase "embryonic stem cells" refers to embryonic cells which are capable of differentiating into cells of all three embryonic germ layers (*i.e.,* endoderm, ectoderm and mesoderm), or remaining in an undifferentiated state. For reference, the phrase "embryonic stem cells" may comprise cells which are obtained from the embryonic tissue formed after gestation (e.g., blastocyst) before implantation of the embryo (*i.e.,* a pre-implantation blastocyst), extended blastocyst cells (EBCs) which are obtained from a post-implantation/pre-gastrulation stage blastocyst (see WO2006/040763) and embryonic germ (EG) cells which are obtained from the genital tissue of a fetus any time during gestation, preferably before 10 weeks of gestation. The embryonic stem cells of some embodiments of the invention can be obtained using well-known cell-culture methods. As a reference example, human embryonic stem cells can be isolated from human blastocysts. Human blastocysts are typically obtained from human *in vivo* preimplantation embryos or from *in vitro* fertilized (IVF) embryos. Alternatively, a single cell human embryo can be expanded to the blastocyst stage. For the isolation of human ES cells the zona pellucida is removed from the blastocyst and the inner cell mass (ICM) is isolated by a procedure in which the trophectoderm cells are lysed and removed from the intact ICM by gentle pipetting. The ICM is then plated in a tissue culture flask containing the appropriate medium which enables its outgrowth. Following 9 to 15 days, the ICM derived outgrowth is dissociated into clumps either by a mechanical dissociation or by an enzymatic degradation and the cells are then re-plated on a fresh tissue culture medium. Colonies demonstrating undifferentiated morphology are individually selected by micropipette, mechanically dissociated into clumps, and re-plated. Resulting ES cells are then routinely split every 4-7 days. For further details on methods of preparation human ES cells see Reubinoff et al Nat Biotechnol 2000, May: 18(5): 559; Thomson et al., [U.S. Pat. No. 5,843,780; Science 282: 1145, 1998; Curr. Top. Dev. Biol. 38: 133, 1998; Proc. Natl. Acad. Sci. USA 92: 7844, 1995]; Bongso et al., [Hum Reprod 4: 706, 1989]; and Gardner et al., [Fertil. Steril. 69: 84, 1998].

It will be appreciated that commercially available stem cells can also be used (for reference only). Human ES cells can be purchased from the NIH human embryonic stem cells registry [Hypertext Transfer Protocol://grants (dot) nih (dot) gov/stem_cells/registry/current (dot) htm]. Non-limiting reference examples of commercially available embryonic stem cell lines are HAD-C102, ESI, BG01, BG02, BG03, BG04, CY12, CY30, CY92, CY10, TE03, TE32, CHB-4, CHB-5, CHB-6, CHB-8, CHB-9, CHB-10, CHB-11, CHB-12, HUES 1, HUES 2, HUES 3, HUES 4, HUES 5, HUES 6, HUES 7, HUES 8, HUES 9, HUES 10, HUES 11, HUES 12, HUES 13, HUES 14, HUES 15, HUES 16, HUES 17, HUES 18, HUES 19, HUES 20, HUES 21, HUES 22, HUES 23, HUES 24, HUES 25, HUES 26, HUES 27, HUES 28, CyT49, RUES3, WA01, UCSF4, NYUES1, NYUES2, NYUES3, NYUES4, NYUES5, NYUES6, NYUES7, UCLA 1, UCLA 2, UCLA 3, WA077 (H7), WA09 (H9), WA13 (H13), WA14 (H14), HUES 62, HUES 63, HUES 64, CT1, CT2, 17 CT3, CT4, MA135, Eneavour-2, WIBR1, WIBR2, WIBR3, WIBR4, WIBR5, WIBR6, HUES 45, Shef 3, Shef 6, BJNhem19, BJNhem20, SA001, SA001.

According to a reference embodiment, the embryonic stem cell line is HAD-C102 or ESI.

In addition, ES cells can be obtained from other species as well, including mouse (Mills and Bradley, 2001), golden hamster [Doetschman et al., 1988, Dev Biol. 127: 224-7], rat [Iannaccone et al., 1994, Dev Biol. 163: 288-92] rabbit [Giles et al. 1993, Mol Reprod Dev. 36: 130-8; Graves & Moreadith, 1993, Mol Reprod Dev. 1993, 36: 424-33], several domestic animal species [Notarianni et al., 1991, J Reprod Fertil Suppl. 43: 255-60; Wheeler 1994, Reprod Fertil Dev. 6: 563-8; Mitalipova et al., 2001, Cloning. 3: 59-67] and non-human primate species (Rhesus monkey and marmoset) [Thomson et al., 1995, Proc Natl Acad Sci U S A. 92: 7844-8; Thomson et al., 1996, Biol Reprod. 55: 254-9].

For reference, e Extended blastocyst cells (EBCs) can be obtained from a blastocyst of at least nine days post fertilization at a stage prior to gastrulation. Prior to culturing the blastocyst, the zona pellucida is digested [for example by Tyrode's acidic solution (Sigma Aldrich, St Louis, MO, USA)] so as to expose the inner cell mass. The blastocysts are then cultured as whole embryos for at least nine and no more than fourteen days post fertilization (*i.e.,* prior to the gastrulation event) *in vitro* using standard embryonic stem cell culturing methods.

Another method for preparing ES cells is described in Chung et al., Cell Stem Cell, Volume 2, Issue 2, 113-117, 7 February 2008. This method comprises removing a single cell from an embryo during an in vitro fertilization process. The embryo is not destroyed in this process.

Yet another method for preparing ES cells is by parthenogenesis. The embryo is also not destroyed in the process.

Currently practiced ES culturing methods are mainly based on the use of feeder cell layers which secrete factors needed for stem cell proliferation, while at the same time, inhibit their differentiation. Exemplary feeder layers include Human embryonic fibroblasts, adult fallopian epithelial cells, primary mouse embryonic fibroblasts (PMEF), mouse embryonic fibroblasts (MEF), murine fetal fibroblasts (MFF), human embryonic fibroblast (HEF), human fibroblasts obtained from the differentiation of human embryonic stem cells, human fetal muscle cells (HFM), human fetal skin cells (HFS), human adult skin cells, human foreskin fibroblasts (HFF), human fibroblasts obtained from the umbilical cord or placenta, and human marrow stromal cells (hMSCs). Growth factors may be added to the medium to maintain the ESCs in an undifferentiated state. Such growth factors include bFGF and/or TGFβ.

Feeder cell free systems have also been used in ES cell culturing, such systems utilize matrices supplemented with serum replacement, cytokines, IL6, soluble IL6 receptor chimera and/or growth factors as a replacement for the feeder cell layer. Stem cells can be grown on a solid surface such as an extracellular matrix (e.g., Matrigel^{RTM} or laminin) in the presence of a culture medium. Unlike feeder-based cultures which require the simultaneous growth of feeder cells and stem cells and which may result in mixed cell populations, stem cells grown on feeder-free systems are easily separated from the surface. The culture medium used for growing the stem cells contains factors that effectively inhibit differentiation and promote their growth such as MEF-conditioned medium and bFGF. However, commonly used feeder-free culturing systems utilize an animal-based matrix (e.g., Matrigel^{RTM}) supplemented with mouse or bovine serum, or with MEF conditioned medium [Xu C, et al. (2001). Feeder-free growth of undifferentiated human embryonic stem cells. Nat Biotechnol. 19: 971-4] which present the risk of animal pathogen cross-transfer to the human ES cells, thus compromising future clinical applications.

Numerous methods are known for differentiating ESCs towards the RPE lineage and include both directed differentiation protocols such as those described in WO 2008/129554, 2013/184809 and spontaneous differentiation protocols such as those described in U.S. Patent No. 8,268,303 and U.S. Patent application 20130196369, .

According to a particular embodiment, the RPE cells are generated from ESC cells using a directed differentiation protocol.

In one exemplary differentiation protocol, the embryonic stem cells are differentiated towards the RPE cell lineage using a first differentiating agent and then further differentiated towards RPE cells using a member of the transforming growth factor-B (TGFβ) superfamily, (e.g. TGFβ1, TGFβ2, and TGFβ3 subtypes, as well as homologous ligands including activin (e.g., activin A, activin B, and activin AB), nodal, anti-mullerian hormone (AMH), some bone morphogenetic proteins (BMP), e.g. BMP2, BMP3, BMP4, BMP5, BMP6, and BMP7, and growth and differentiation factors (GDF)). According to a specific embodiment, the member of the transforming growth factor-B (TGFβ) superfamily is activin A - e.g. between 20-200 ng/ml e.g. 100-180 ng/ml.

According to a particular embodiment, the first differentiating agent is nicotinamide (NA) - e.g. between 1-100 mM, 5-50 mM, 5-20 mM, e.g. 10 mM.

NA, also known as "niacinamide", is the amide derivative form of Vitamin B3 (niacin) which is thought to preserve and improve beta cell function. NA has the chemical formula C₆H₆N₂O. NA is essential for growth and the conversion of foods to energy, and it has been used in arthritis treatment and diabetes treatment and prevention.

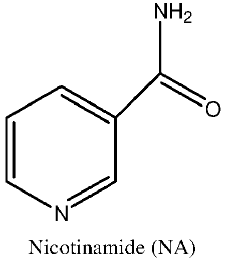

In the context of the present disclosure, the term NA also denotes derivatives of NA and nicotinamide mimics. The term "derivative of nicotinamide (NA)" as used herein denotes a compound which is a chemically modified derivative of the natural NA. The chemical modification may include, for example, a substitution on the pyridine ring of the basic NA structure (via the carbon or nitrogen member of the ring), via the nitrogen or the oxygen atoms of the amide moiety, as well as deletion or replacement of a group, e.g. to form a thiobenzamide analog of NA, all of which being as appreciated by those versed in organic chemistry. The derivative in the context of the invention also includes the nucleoside derivative of NA (e.g. nicotinamide adenine). A variety of derivatives of NA are described, some also in connection with an inhibitory activity of the PDE4 enzyme (WO03/068233; WO02/060875; GB2327675A), or as VEGF-receptor tyrosine kinase inhibitors (WO01/55114). For example, the process of preparing 4-aryl-nicotinamide derivatives (WO05/014549).

Nicotinamide mimics may be substituted for nicotinamide in the media. Nicotinamide mimics encompass any compound which recapitulates the effects of nicotinamide in the differentiation and maturation of RPE cells from pluripotent cells.

Nicotinamide mimics include modified forms of nicotinamide, and chemical analogs of nicotinamide. Exemplary nicotinamide mimics include benzoic acid, 3-aminobenzoic acid, and 6-aminonicotinamide. Another class of compounds that may act as nicotinamide mimics are inhibitors of poly(ADP-ribose) polymerase (PARP). Exemplary PARP inhibitors include 3-aminobenzamide, Iniparib (BSI 201), Olaparib (AZD-2281), Rucaparib (AG014699, PF- 01367338), Veliparib (ABT-888), CEP 9722, MK 4827, and BMN-673.

According to a particular embodiment, the differentiation is effected as follows:
a) culture of ESCs in a medium comprising a first differentiating agent (e.g. nicotinamide); and
b) culture of cells obtained from step a) in a medium comprising a member of the TGFβ superfamily (e.g. activin A) and the first differentiating agent (e.g. nicotinamide).

Preferably step (a) is effected in the absence of the member of the TGFβ superfamily.

The above described protocol may be continued by culturing the cells obtained in step b) in a medium comprising the first differentiating agent (e.g. nicotinamide), but devoid of a member of the TGFβ superfamily (e.g. activin A). This step is referred to herein as step (c).

The above described protocol is now described in further detail, with additional embodiments.

Step (a): The differentiation process is started once sufficient quantities of ESCs are obtained. They are typically removed from the adherent cell culture (e.g. by using collagenase A, dispase, TrypLE select, EDTA) and plated onto a non-adherent substrate (e.g. cell culture plate) in the presence of nicotinamide (and the absence of activin A). Once the cells are plated onto the non-adherent substrate (e.g. cell culture plate), the cell culture may be referred to as a cell suspension, preferably free floating clusters in a suspension culture, i.e. aggregates of cells derived from human embryonic stem cells (hESCs). Sources of free floating stem cells were previously described in WO 06/070370

This stage may be effected for a minimum of 1 day, more preferably two days, three days, 1 week or even 10 days. Preferably, the cells are not cultured for more than 2 weeks in suspension together with the nicotinamide (and in the absence of activin).

According to a preferred embodiment, when the cells are cultured on the non-adherent substrate e.g. cell culture plates, the atmospheric oxygen conditions are manipulated such that the percentage is less than about 20 %, 15 %, 10 %, more preferably less than about 9 %, less than about 8 %, less than about 7 %, less than about 6 % and more preferably about 5 %.

According to a particular embodiment, the cells are cultured on the non-adherent substrate initially under normal atmospheric oxygen conditions and then lowered to less than normal atmospheric oxygen conditions.

Examples of non-adherent substrates include but are not limited to fibronectin, laminin, polyD-lysine and gelatin.

Examples of non-adherent cell culture plates include those manufactured by Hydrocell (e.g. Cat No. 174912), Nunc etc.

Step (b): Following the first stage of directed differentiation, (step a; i.e. culture in the presence of nicotinamide (e.g. 10 mM) under non-adherent culture conditions under low or normal oxygen atmospheric conditions), the semi-differentiated cells are then subjected to a further stage of differentiation on an adherent substrate - culturing in the presence of nicotinamide (e.g. 10 mM) and activin A (e.g. 140 ng/ml, 150 ng/ml, 160 ng/ml or 180 ng/ml). This stage may be effected for at least one day, at least two days, at least three days, at least 5 days, at least one week, at least two weeks, at least three weeks, at least four weeks, at least five weeks, at least six weeks, at least seven weeks, at least eight weeks, at least nine weeks, at least ten weeks. Preferably this stage is effected for about two weeks. This stage of differentiation may be effected at low or normal atmospheric oxygen conditions, as detailed herein above.

Step (c): Following the second stage of directed differentiation (i.e. culture in the presence of nicotinamide and activin A on an adherent substrate; step (b)), the further differentiated cells are optionally subjected to a subsequent stage of differentiation on the adherent substrate - culturing in the presence of nicotinamide (e.g. 10 mM), in the absence of activin A. This stage may be effected for at least one day, 2, days, 5 days, at least one week, at least two weeks, at least three weeks or even four weeks. Preferably this stage is effected for about one week. This stage of differentiation may also be carried out at low or normal atmospheric oxygen conditions, as detailed herein above.

Following this differentiation step, the atmospheric oxygen conditions may optionally be returned to normal atmospheric conditions and cultured for at least one more day, at least 2 more days, at least 5 more days at least one more week (e.g. up to two weeks) in the presence of nicotinamide (e.g. 10 mM) and in the absence of activin A.

The basic medium in accordance with the description is any known cell culture medium known in the art for supporting cells growth *in vitro,* typically, a medium comprising a defined base solution, which includes salts, sugars, amino acids and any other nutrients required for the maintenance of the cells in the culture in a viable state. Non-limiting examples of commercially available basic media that may be utilized in accordance with the description comprise Nutristem (without bFGF and TGFβ for ESC differentiation, with bFGF and TGFβ for ESC expansion) Neurobasal™, KO-DMEM, DMEM, DMEM/F12, Cellgro™ Stem Cell Growth Medium, or X-Vivo™. The basic medium may be supplemented with a variety of agents as known in the art dealing with cell cultures. The following is a non-limiting reference to various supplements that may be included in the culture system to be used in accordance with the present disclosure:
- serum or with a serum replacement containing medium, such as, without being limited thereto, knock out serum replacement (KOSR), Nutridoma-CS, TCH™, N2, N2 derivative, or B27 or a combination;
- an extracellular matrix (ECM) component, such as, without being limited thereto, fibronectin, laminin, collagen and gelatin. The ECM may them be used to carry the one or more members of the TGFβ superfamily of growth factors;
- an antibacterial agent, such as, without being limited thereto, penicillin and streptomycin;
- non-essential amino acids (NEAA),
   neurotrophins which are known to play a role in promoting the survival of SCs in culture, such as, without being limited thereto, BDNF, NT3, NT4.

According to a preferred embodiment, the medium used for differentiating the ESCs is Nutristem medium (Biological Industries, 05-102-1A or 05-100-1A).

According to a particular embodiment differentiation of ESCs is effected under xeno free conditions.

According to one embodiment, the proliferation/growth medium is devoid of xeno contaminants i.e. free of animal derived components such as serum, animal derived growth factors and albumin. Thus, according to this embodiment, the culturing is performed in the absence of xeno contaminants.

Other methods for culturing ESCs under xeno free conditions are provided in U.S. Patent Application No. 201301963 69.

The preparations comprising RPE cells may be prepared in accordance with Good Manufacturing Practices (GMP) (e.g., the preparations are GMP-compliant) and/or current Good Tissue Practices (GTP) (e.g., the preparations may be GTP-compliant).

During differentiation steps, the embryonic stem cells may be monitored for their differentiation state. Cell differentiation can be determined upon examination of cell or tissue-specific markers which are known to be indicative of differentiation.

Tissue/cell specific markers can be detected using immunological techniques well known in the art [Thomson JA et al., (1998). Science 282: 1145-7]. Examples include, but are not limited to, flow cytometry for membrane-bound or intracellular markers, immunohistochemistry for extracellular and intracellular markers and enzymatic immunoassay, for secreted molecular markers (e.g. PEDF).

Thus, according to another aspect of the present description there is provided a method of generating retinal epithelial cells comprising:
(a) culturing pluripotent stem cells in a medium comprising a differentiating agent so as to generate differentiating cells, wherein the medium is devoid of a member of the transforming growth factor β (TGF β) superfamily;
(b) culturing the differentiating cells in a medium comprising the member of the transforming growth factor β (TGF β) superfamily and the differentiating agent to generate cells which are further differentiated towards the RPE lineage;
(c) analyzing the secretion of Pigment epithelium-derived factor (PEDF) from the cells which are further differentiated towards the RPE lineage; and
(d) culturing the cells which are further differentiated towards the RPE lineage in a medium comprising a differentiating agent so as to generate RPE cells, wherein the medium is devoid of a member of the transforming growth factor β (TGF β) superfamily, wherein step (d) is effected when the amount of the PEDF is above a predetermined level.

Preferably, step (d) is effected when the level of PEDF is above 100 ng/ml/day, 200 ng/ml/day, 300 ng/ml/day, 400 ng/ml/day, or 500 ng/ml/day.

Another method for determining potency of the cells during or following the differentiation process is by analyzing barrier function and polarized PEDF and VEGF secretion.

Once the cells are promoted into the RPE fate, the RPE cells may be selected and/or expanded.

According to a particular embodiment, the selection is based on a negative selection - i.e. removal of non-RPE cells. This may be done mechanically by removal of non-pigmented cells or by use of surface markers.

According to another embodiment, the selection is based on a positive selection i.e. selection of pigmented cells. This may be done by visual analysis or use of surface markers.

According to still another embodiment, the selection is based first on a negative selection and then on a positive selection.

Expansion of RPE cells may be effected on an extra cellular matrix, e.g. gelatin or collagen, laminin and poly-D-lysine. For expansion, the cells may be cultured in serum-free KOM, serum comprising medium (e.g. DMEM + 20 %) or Nutristem medium (06-5102-01-1A Biological Industries). Under these culture conditions, the pigmented cells reduce pigmentation and acquire a fibroid-like morphology. Following further prolonged culture and proliferation into high-density cultures, the cells reacquire the characteristic polygonal shape morphology and increase pigmentation of RPE cells.

The RPE cells may be expanded in suspension or in a monolayer. The expansion of the RPE cells in monolayer cultures may be modified to large scale expansion in bioreactors by methods well known to those versed in the art.

The population of RPE cells generated according to the methods described herein may be characterized according to a number of different parameters.

Thus, for example, the RPE cells obtained are polygonal in shape and are pigmented.

According to one embodiment, at least 95 %, at least 96 %, at least 97 %, at least 98 %, at least 99 % or even 100 % of the cells of the RPE cell populations obtained co-express both premelanosome protein (PMEL17) and cellular retinaldehyde binding protein (CRALBP).

According to a particular embodiment, the cells coexpress PMEL17 (SwissProt No. P40967) and at least one polypeptide selected from the group consisting of cellular retinaldehyde binding protein (CRALBP; SwissProt No. P12271), lecithin retinol acyltransferase (LRAT; SwissProt No. 095327) and sex determining region Y-box 9 (SOX 9; P48436).

According to a particular embodiment, at least 80 % of the cells of the population express detectable levels of PMEL17 and one of the above mentioned polypeptides (e.g. CRALBP), more preferably at least 85 % of the cells of the population express detectable levels of PMEL17 and one of the above mentioned polypeptides (e.g. CRALBP), more preferably at least 90 % of the cells of the population express detectable levels of PMEL17 and one of the above mentioned polypeptides (e.g. CRALBP), more preferably at least 95 % of the cells of the population express detectable levels of PMEL17 and one of the above mentioned polypeptides (e.g. CRALBP), more preferably 100 % of the cells of the population express detectable levels of PMEL17 and one of the above mentioned polypeptides (e.g. CRALBP as assayed by a method known to those of skill in the art (e.g. FACS).

According to another embodiment, the level of CRALBP and one of the above mentioned polypeptides (e.g. PMEL17) coexpression (e.g. as measured by the mean fluorescent intensity) is increased by at least two fold, more preferably at least 3 fold, more preferably at least 4 fold and even more preferably by at least 5 fold, at least 10 fold, at least 20 fold, at least 30 fold, at least 40 fold, at least 50 as compared to non-differentiated ESCs.

In one embodiment, the RPE are terminally differentiated) and do not express Pax6.

In another embodiment, the RPE cells are terminally differentiated and express Pax6.

The RPE cells described herein may also act as functional RPE cells after transplantation where the RPE cells form a monolayer between the neurosensory retina and the choroid in the patient receiving the transplanted cells. The RPE cells may also supply nutrients to adjacent photoreceptors and dispose of shed photoreceptor outer segments by phagocytosis.

According to one embodiment, the trans-epithelial electrical resistance of the cells in a monolayer is greater than 100 ohms.

Preferably, the trans-epithelial electrical resistance of the cells is greater than 150, 200, 250, 300, 300, 400, 500, 600, 700, 800 or even greater than 900 ohms.

Devices for measuring trans-epithelial electrical resistance (TEER) are known in the art and include for example EVOM2 Epithelial Voltohmmeter, (World Precision Instruments).

It will be appreciated that the cell populations disclosed herein are devoid of undifferentiated human embryonic stem cells. According to one embodiment, less than 1:250,000 cells are Oct4⁺TRA-1-60⁺ cells, as measured for example by FACS. The cells also have down regulated (by more than 5,000 fold) expression of GDF3 or TDGF as measured by PCR.

The RPE cells of this aspect of the present invention do not express embryonic stem cell markers. Said one or more embryonic stem cell markers may comprise OCT-4, NANOG, Rex- 1, alkaline phosphatase, Sox2, TDGF- beta, SSEA-3, SSEA-4, TRA-1 -60, and/or TRA- 1-81.

The RPE preparations may be substantially purified, with respect to non-RPE cells, comprising at least about 75%, 80%, 85%, 90%, 91 %, 92%, 93%, 94%, 95%, 96%, 97%, 98%, 99%, or 100% RPE cells. The RPE cell preparation may be essentially free of non-RPE cells or consist of RPE cells. For example, the substantially purified preparation of RPE cells may comprise less than about 25%, 20%, 15%, 10%, 9%, 8%, 7%, 6%, 5%, 4%, 3%, 2%, or 1% non-RPE cell type. For example, the RPE cell preparation may comprise less than about 25%, 20%, 15%, 10%, 9%, 8%, 7%, 6%, 5%, 4%, 3%, 2%, 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% non-RPE cells.

The RPE cell preparations may be substantially pure, both with respect to non-RPE cells and with respect to RPE cells of other levels of maturity. The preparations may be substantially purified, with respect to non-RPE cells, and enriched for mature RPE cells. For example, in RPE cell preparations enriched for mature RPE cells, at least about 30%, 40%, 45%, 50%, 55%, 60%, 65%, 70%, 75%, 80%, 85%, 90%, 91%, 92%, 93%, 94%, 95%, 96%, 97%, 98%, 99%, 99%, or 100% of the RPE cells are mature RPE cells. The preparations may be substantially purified, with respect to non-RPE cells, and enriched for differentiated RPE cells rather than mature RPE cells. For example, at least about 30%, 40%, 45%, 50%, 55%, 60%, 65%, 70%, 75%, 80%, 85%, 90%, 91%, 92%, 93%, 94%, 95%, 96%, 97%, 98%, 99%, or 100% of the RPE cells may be differentiated RPE cells rather than mature RPE cells.

The preparations described herein may be substantially free of bacterial, viral, or fungal contamination or infection, including but not limited to the presence of HIV I, HIV 2, HBV, HCV, HAV, CMV, HTLV 1, HTLV 2, parvovirus B19, Epstein-Barr virus, or herpesvirus 1 and 2, SV40, HHV5, 6, 7, 8, CMV, polyoma virus, HPV, Enterovirus. The preparations described herein may be substantially free of mycoplasma contamination or infection.

Another way of characterizing the cell populations disclosed herein is by marker expression. Thus, for example, at least 80 %, 85 %, 90 %, 95 % or 100 % of the cells express Bestrophin 1, as measured by immunostaining. According to one embodiment, between 85-100 % of the cells express bestrophin.

According to another embodiment, at least 80 %, 85 %, 87 %, 89 %, 90 %, 95%, 97 % or 100 % of the cells express Microphthalmia-associated transcription factor (MITF), as measured by immunostaining. For example, between 85-100% of the cells express MITF.

According to another embodiment, at least 80 % 85 %, 87 %, 89 %, 90 %, 95%, 97 % or 100 % of the cells express paired box gene 6 (PAX-6) as measured by FACS.

The cells described herein can also be characterized according to the quantity and/or type of factors that they secrete. Thus, according to one embodiment, the cells preferably secrete more than 500, 1000, 2000, 3000 or even 4000 ng of Pigment epithelium-derived factor (PEDF) per ml per day, as measured by ELISA. According to a particular embodiment, the cells secrete between about 1250-4000 ng of Pigment epithelium-derived factor (PEDF) per ml per day.

It will be appreciated that the RPE cells generated herein secrete PEDF and vascular endothelial growth factor (VEGF) in a polarized manner. According to particular embodiments, the ratio of apical secretion of PEDF: basal secretion of PEDF is greater than 1. According to particular embodiments, the ratio of apical secretion of PEDF: basal secretion of PEDF is greater than 2. According to particular embodiments, the ratio of apical secretion of PEDF: basal secretion of PEDF is greater than 3, greater than 4, greater than 5, greater than 6, greater than 7 or even greater than 8. In addition, the ratio of basal secretion of VEGF: apical secretion of VEGF is greater than 1. According to particular embodiments, the ratio of basal secretion of VEGF: apical secretion of VEGF is greater than 1.5, 2 or 2.5.

The stability of the cells is another characterizing feature. Thus, for example the amount of PEDF secretion remains stable in the cells following a 6 hour, 8 hour, 10 hour, 12 hour or even 24 hour incubation at 2-8 °C (when the cells are formulated in BSS + buffer). Further, the polarized secretion of PEDF and VEGF remains stable following a 6 hour, 8 hour, 10 hour, 12 hour or even 24 hour incubation at 2-8 °C (when the cells are formulated in BSS + buffer). Further, the TEER of the cells remains stable in the cells following a 6 hour, 8 hour, 10 hour, 12 hour or even 24 hour incubation at 2-8 °C (when the cells are formulated in BSS + buffer).

In another embodiment, the cells are characterized by their therapeutic effect. Thus, for example the present inventors have shown that the cell populations are capable of rescuing visual acuity in the RCS rat following subretinal administration. In addition, the cell populations are capable of rescuing photoreceptors (e.g. cone photoreceptors) for up to 180 days post-subretinal administration in the RCS rat.

It would be well appreciated by those versed in the art that the derivation of RPE cells is of great benefit. They may be used as an *in vitro* model for the development of new drugs to promote their survival, regeneration and function. RPE cells may serve for high throughput screening for compounds that have a toxic or regenerative effect on RPE cells. They may be used to uncover mechanisms, new genes, soluble or membrane-bound factors that are important for the development, differentiation, maintenance, survival and function of photoreceptor cells.

The RPE cells may also serve as an unlimited source of RPE cells for transplantation, replenishment and support of malfunctioning or degenerated RPE cells in retinal degenerations. Furthermore, genetically modified RPE cells may serve as a vector to carry and express genes in the eye and retina after transplantation.

The RPE cells produced by the method of the present disclosure may be used for large scale and/or long term cultivation of such cells. To this end, the method of the invention is to be performed in bioreactors suitable for large scale production of cells, and in which undifferentiated hSCs are to be cultivated in accordance with the invention. General requirements for cultivation of cells in bioreactors are well known to those versed in the art.

Harvesting of the cells may be performed by various methods known in the art. Non-limiting examples include mechanical dissection and dissociation with papain or trypsin (e.g. TrypLE select). Other methods known in the art are also applicable.

"Effective amount," as used herein, refers broadly to the amount of a compound or cells that, when administered to a patient for treating a disease, is sufficient to effect such treatment for the disease. The effective amount may be an amount effective for prophylaxis, and/or an amount effective for prevention. The effective amount may be an amount effective to reduce, an amount effective to prevent the incidence of signs/symptoms, to reduce the severity of the incidence of signs/symptoms, to eliminate the incidence of signs/symptoms, to slow the development of the incidence of signs/symptoms, to prevent the development of the incidence of signs/symptoms, and/or effect prophylaxis of the incidence of signs/symptoms. The "effective amount" may vary depending on the disease and its severity and the age, weight, medical history, susceptibility, and preexisting conditions, of the patient to be treated. The term "effective amount" is synonymous with "therapeutically effective amount" for purposes of this disclosure.

It is expected that during the life of a patent maturing from this application many relevant technologies will be developed for the generation of RPE cells, and the term RPE cells is intended to include all such new technologies *a priori.*

As used herein the term "about" refers to ± 10 %.

The terms "comprises", "comprising", "includes", "including", "having" and their conjugates means "including but not limited to".

The term "consisting of' means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following reference examples.

### REFERENCE EXAMPLES

Reference is now made to the following reference examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### REFERENCE EXAMPLE 1

### Device of delivery

***Study objective:*** to compare the performance of two retinal cannulas: MedOne PolyTip 25 gauge (G) needles with 38G flexible cannula to Peregrine 25 (G) needle with 41 G flexible cannula.

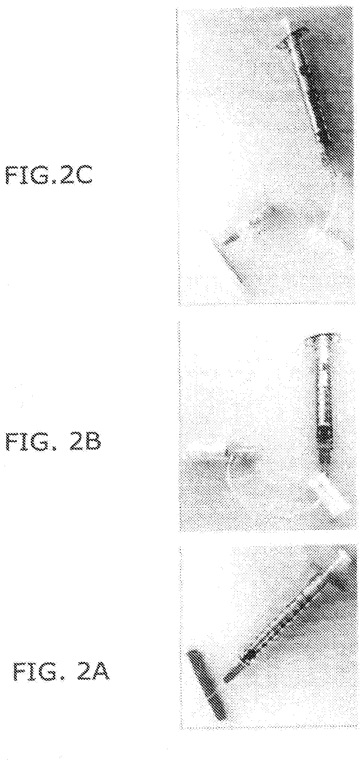

***Experimental Design:*** Cryopreserved vials of RPE cells (1.5 x10⁶cells/vial/1 ml frozen cell suspension) were thawed, contents were transferred gently to DMEM containing 20 % human serum, filtered, washed again with DMEM containing 20 % human serum, and then washed with BSS Plus. The cell pellet was then resuspended in 0.5-1 ml BSS Plus and viable cell enumeration by Trypan Blue exclusion was carried out. The cells were resuspended at different cell densities that following injection through the device of delivery would yield the intended Phase I/IIa clinical doses of 50 x10³, 200x10³ or 500 x10³ viable cells per 100-150 µl BSS Plus. Prior to loading into the device of delivery, the appearance of formulated RPE cells was tested to verify absence of visible foreign particles. RPE cells in their final formulation were loaded into a sterile 1 mL syringe using a sterile 18G needle (Figure 2A). The 18G needle was then replaced with a sterile 10 cm extension tube (Figure 2B), air was removed through the extension tube, and a sterile 25G/38G cannula (MedOne) or a 25G/41G cannula (Peregrine) was attached to the end of the extension tube (Figure 2C). Air was then removed from the cannula until a drop of cells appeared at the tip of the cannula. According to one option, the plunger is pushed back and 10-20 µl of air is inserted into the end of the cannula.

The RPE cells were delivered in fractions of 100-150 µl at an approximate rate of 50 µl/minute. Cell concentration, volume, viability post-delivery and delivery rate (volume/min) were determined in each study and compared.

Vitality and functional activity of the RPE cells prior and post-delivery through the device were determined.

Reproducibility of the actual cell dose delivered per injected was assessed for each of the intended clinical doses (i.e. 50 x10³, 200x10³ or 500 x10³ viable cells per 100-150 µl BSS Plus) by 3 testers.

### RESULTS

The inner and outer diameters of the MedOne cannula are 99 µm and 120 µm respectively. The retinal cannul Peregrine 25 (G) needle with 41G flexible cannula has a smaller inner and outer diameter of 71 µm and 96.5 µm respectively. Similar cell recovery was seen with the MedOne and the Peregrine cannulas (see Table 1 herein below).

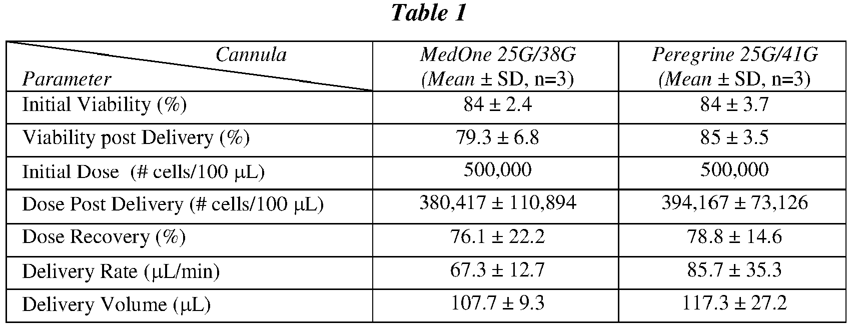

### Determination of the Initial cell dose for recovery of the high, medium and low clinical doses using Peregrine 25G/41G:

According to the data above, the recovery of the high clinical cell dose (500,000 cells/100µl) with Peregrine 25G/41G was 78.8 +/- 14.6 % (mean +/- SD). To yield the intended high clinical cell dose of 500,000 cells /100µl, several initial cell doses were tested of which an initial cell dose of 700,000 cells/100µl that yielded 507,296 +/-81,803 cells/100µl (mean +/- SD, n=6, 3 testers) was selected for use. Table 2 below summarizes the data obtained by 3 different testers.

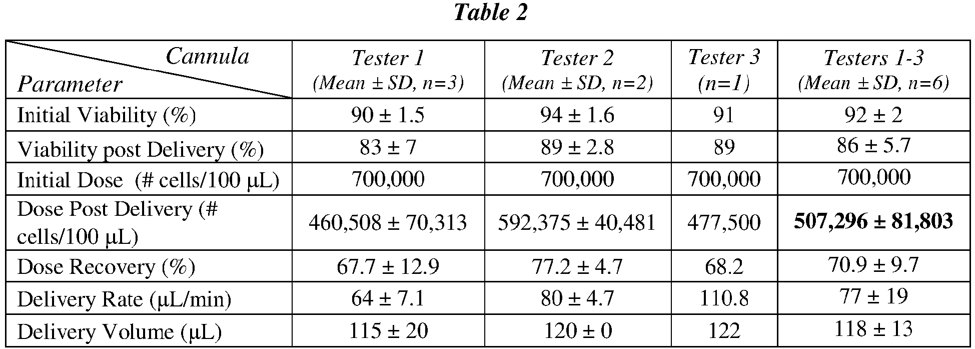

To yield the intended mid clinical cell dose of 200,000 cells /100µl, an initial cell dose of 270,000 cels/100µl (range 247,000-292,000 cells/100µl) that yielded 191,250 +/- 67,511 cells/100µl (mean +/- SD, n=6, 3 testers) was selected for use. Table 3 below summarizes the data obtained by 3 different testers.

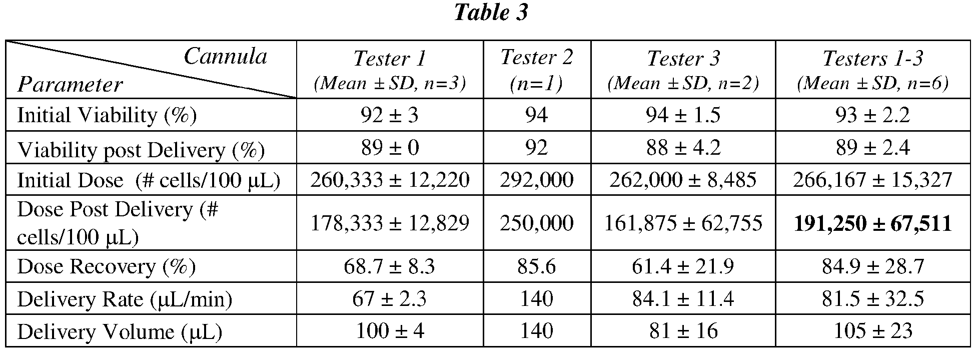

To yield the intended low clinical cell dose of 50,000 cells /100µl, an initial cell dose of 70,000 cells/100µl that yielded 50,688 +/- 6,533 cells/100µl (mean +/- SD, n=5, 3 testers) was selected for use. Table 4 below summarizes the data obtained by 3 different testers.

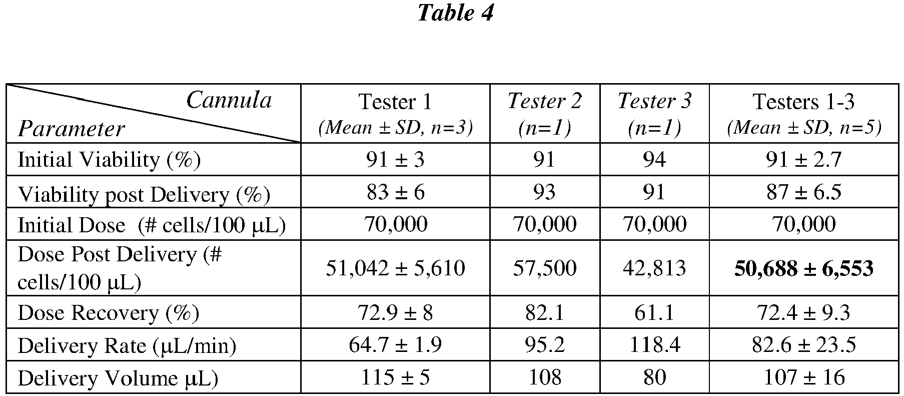

### RPE Potency Post Delivery through Peregrine 25G/41G:

To qualify the Peregrine 26G/41G cannula, the potency of the RPE cells formulated at the high initial clinical dose of 700,000 cells/100µl was tested post-delivery. Potency was assessed using the polarization assay in which the barrier function of the RPE cells as well as the ability to secrete Pigment Epithelium Derived Factor (PEDF) and Vascular Endothelial Derived Factor (VEGF) in a polarized manner were tested using the transwell system. As can be seen in Table 5A, barrier function/trans-epithelial electrical resistance (TEER) and polarized secretion of PEDF and VEGF were similar prior and post delivery. Viability and cell concentration post delivery were within the expected range (Table 5B).

**Table 5A**

| *Experiment # 47* | *PEDF day 14 (ng*/*mL*/*day)* | *TEER (*Ω*)* | *PEDF Apical*/*Basal* | *VEGF Basal*/*Apical* |
|---|---|---|---|---|
| Prior to Delivery | 1,501 | 384 | 4.74 | 2.58 |
| Post Deliver | 1,812 | 492 | 4.85 | 2.99 |
| OpRegen 5C Control | 1,858 | 314 | 4.3 | 2.61 |

**Table 5B**

| *Outcome Measure* | *Test Results* |
|---|---|
| Initial Viability (%) | 95 |
| Viability post Delivery (%) | 91 |
| Initial Dose (# cells/100 µL) | 700,000 |
| Dose Post Delivery (# cells/100 µL) | 621,000 |
| Dose Recovery (%) | 73.9 |
| Delivery Rate (µL/min) | 76.6 |
| Delivery Volume (µL) | 120 |

In addition to maintained polarization ability, the RPE cell vitality post-delivery was preserved (data not shown).

The data presented herein above support the use of the Peregrine 25G/41G cannula at the intended clinical doses of 50,000, 200,000 and 500,000 cells per 100 µl. To reach these final clinical doses, RPE cells in a final concentration of 70,000, 270,000 and 700,000 viable cells per 100 µl, respectively may be prepared.

### Determination of the Initial RPE Cell Dose for Recovery of the Low Clinical Dose of 50,000 Cells /50 µl:

A cell dose of 70,000 RPE cells/50 µL was initially tested for the ability to yield the intended low clinical cell dose of 50,000 cells/50 µL. As shown in Table 6, an initial cell dose of 70,000 cells/50 µL yielded 38,697 ± 5,505 cells/50 µL (mean ± SD, n=3, 3 testers) was prepared. Since the average recovery was 55% ± 7.8% (mean ± SD, n=3, 3 testers), and since the intended dose of 50,000 cells/50 µL was not reached, an initial cell density of 100,000 cells/50 µL was tested in the second set of experiments. As shown in Table 7, an initial cell dose of 100,000 cells/50 µL yielded 62,517 ± 4,625 cells/50 µL (mean ± SD, n=3, 3 testers; OpRegen® Batch 5D). The average recovery was 61% ± 4.5% (mean ± SD, n=3, 3 testers).

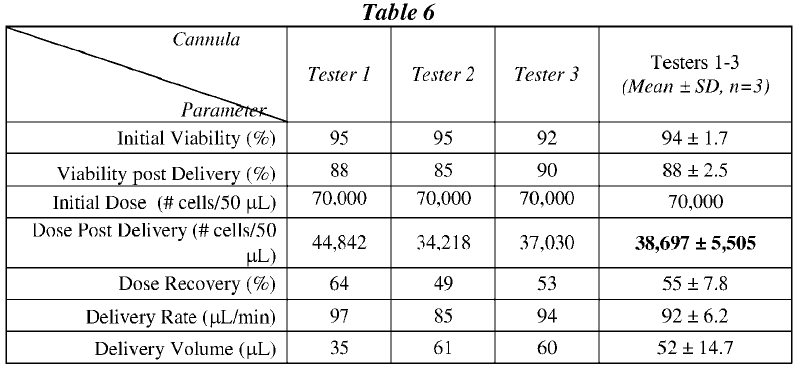

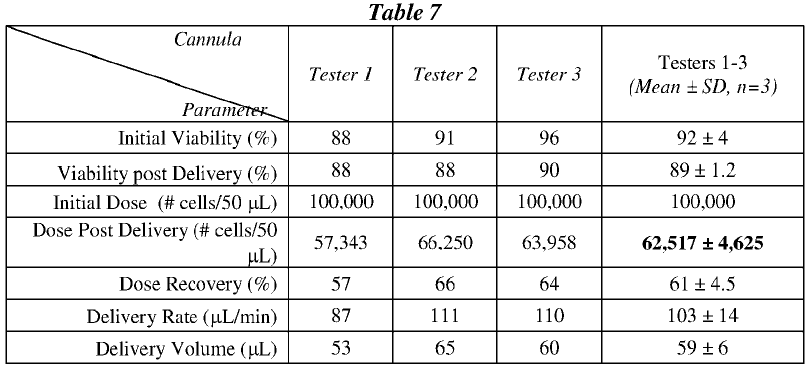

### REFERENCE EXAMPLE 2

### Clinical Experiment

***Study Design:*** Single center Phase I/IIa study of 15 patients with advanced dry form AMD and geographic atrophy (GA) divided into four cohorts: the first 3 cohorts, each consisting of 3 legally blind patients with best corrected visual acuity of 20/200 or less, will receive a single subretinal injection of RPE cells, using sequentially escalating dosages of 50x10³, 200x10³, and 500x10³ cells per cohort, respectively. The fourth cohort will include 6 patients with best corrected visual acuity of 20/100 or less, who will receive a single subretinal injection of 500,000 RPE cells. Staggering intervals within and between cohorts will be applied.

Following a vitrectomy, cells will be delivered into the subretinal space in the macular area via a cannula through a small retinotomy. A total volume of up to 50-150 µl cell suspension will be injected in areas at risk for GA expansion.

Along with the surgical procedure, patients will receive light immunosuppression and antibiotic treatment, consisting of the following:
1. Topical steroidal and antibiotic treatment as customary following vitrectomy: A course of topical steroid therapy (Predforte drops 4-8 times daily, with gradual taper) and topical antibiotic drops (Oflox or equivalent 4 times daily) over the course of 6 weeks.
2. Systemic (PO) Tacrolimus 0.01 mg/kg daily (dose will be adjusted to reach blood concentration of 3-7 ng/ml), from a week before transplantation and continued until 6 weeks post transplantation.
3. Systemic (PO) Mycophenolate mofetil, total 2 gr/day, given from 2 weeks before transplantation and continued for one year post transplantation.

Patients will be assessed at pre-scheduled intervals throughout the 12 months following the administration of the cells. Post study follow-up will occur at 15 months, 2, 3, 4 and 5 years post-surgery. In patients who develop side effects related to the immunosuppressive treatment, an attempt will be made to control these side effects (for example, improve blood pressure control if hypertension develops). In the case of uncontrollable side effects, treatment with the causative immunosuppressive agent will be modified in consultation with the study internist.

### Inclusion Criteria:

1. Age 55 and older;
2. Diagnosis of dry (non-neovascular) age related macular degeneration in both eyes;
3. Funduscopic findings of dry AMD with geographic atrophy in the macula, above 0.5 disc area (1.25mm² and up to 17 mm²) in size in the study eye and above 0.5 disc area in the fellow eye;
4. Best corrected central visual acuity equal or less than 20/200 in cohorts 1-3 and equal or less than 20/100 in cohort 4 in the study eye by ETDRS vision testing;
5. Vision in the non-operated eye must be better than or equal to that in the operated eye;
6. Patients with sufficiently good health to allow participation in all study-related procedures and complete the study (medical records);
7. Ability to undergo a vitreoretinal surgical procedure under monitored anesthesia care;
8. Normal blood counts, blood chemistry, coagulation and urinalysis;
9. Negative for HIV, HBC, and HCV, negative for CMV IgM and EBV IgM;
10. Patients with no current or history of malignancy (with the exception of successfully treated basal/squamous cell carcinoma of the skin) based on age matched screening exam (at discretion of the study physician);
11. Patients allowed to discontinue taking aspirin, aspirin-containing products and any other coagulation-modifying drugs, 7 days prior to surgery;
12. Willing to defer all future blood and tissue donation;
13. Able to understand and willing to sign informed consent.

### Exclusion Criteria:

1. Evidence of neovascular AMD by history, as well as by clinical exam, fluorescein angiography (FA), or ocular coherence tomography (OCT) at baseline in either eye;
2. History or presence of diabetic retinopathy, vascular occlusions, uveitis, Coat's disease, glaucoma, cataract or media opacity preventing posterior pole visualization or any significant ocular disease other than AMD that has compromised or could compromise vision in the study eye and confound analysis of the primary outcome;
3. History of retinal detachment repair in the study eye;
4. Axial myopia greater than -6 diopters;
5. Ocular surgery in the study eye in the past 3 months;
6. History of cognitive impairments or dementia;
7. Contraindication for systemic immunosuppression;
8. History of any condition other than AMD associated with choroidal neovascularization in the study eye (e.g. pathologic myopia or presumed ocular histoplasmosis);
9. Active or history for the following diseases: cancer, renal disease, diabetes, myocardial infraction in previous 12 months, immunodeficiency;
10. Female; pregnancy or lactation;
11. Current participation in another clinical study. Past participation (within 6 months) in any clinical study of a drug administered systemically or to the eye.

The safety and tolerability of the surgical procedure and the safety of the cell graft will be assessed separately. Assessment of surgical safety will include the following measures:
1. Unhealing retinal detachment
2. Proliferative vitreo-retinopathy (PVR)
3. Subretinal, retinal or intravitreal hemorrhage
4. Injury to relatively still healthy retina at the site of surgery

The safety and tolerability of the cell graft will be evaluated using the following adverse events that will be graded according to the National Cancer Institute (NCI) grading system:
1. Teratoma and/or tumor and/or ectopic tissue formation
2. Infection
3. Uveitis, Vasculitis or PVR
4. Accelerated progression of GA
5. Progression to neovascular AMD in the study eye
6. Serious inflammatory reaction against the allotransplanted cells

The secondary exploratory efficacy endpoints will be measured by duration of graft survival and by the examination of the following:
1. Rate of GA progression
2. Retinal sensitivity in engrafted regions, extent and depth of central scotomata
3. Changes in visual acuity

### Surgical Procedure:

The eye chosen for RPE administration will be the eye with worse visual function. The surgery will be performed by retro-bulbar or peri-bulbar anesthetic block accompanied by monitored intravenous sedation or by general anesthesia, at the discretion of the surgeon and in discussion with the patient. The eye undergoing surgery will be prepped and draped in sterile fashion according to the institution protocol. After the placement of a lid speculum, a standard 3-port vitrectomy will be performed. This will include the placement of a 23G infusion cannula and two 23G ports. After visual inspection of the infusion cannula in the vitreous cavity, the infusion line will be opened to ensure that structure of the eye globe is maintained throughout the surgery. A careful core vitrectomy will then be performed with standard 23G instruments, followed by detachment of the posterior vitreous face. This will allow unobstructed access to the posterior pole.

RPE will be introduced into the subretinal space at a predetermined site within the posterior pole, preferably penetrating the retina in an area that is still relatively preserved close to the border of GA. Blood vessels will be avoided. The cells will be delivered to the subretinal space via formation of a small bleb, with a volume of 50-150 µl.

The delivery system is composed of a 1 mL syringe that through a 10 cm extension tube is connected to a Peregrine 25G/41G flexible retinal cannula.

Any cells that refluxed into the vitreal space will be removed and fluid-air exchange will be performed. Prior to removal of the infusion cannula, careful examination will be performed to ensure that no iatrogenic retinal tears or breaks were created. The infusion cannula will then be removed. Subconjunctival antibiotics and steroids will be administered. The eye will be covered with a patch and plastic shield. The surgical administration procedure will be recorded.

***Dose:*** A low dose of 50,000 cells/50 µL or 50,000 cells/100 µL, medium dose of 200,000 cells/100 µL and a high dose of 500,000 cells/100 µL will be used. Dose selection was based on the safety of the maximal feasible dose tested in preclinical studies and the human equivalent dose calculated based on eye and bleb size.

### End-point parameters:

### 1. Safety of Surgical Procedure:

Persistent/recurrent retinal detachment
Proliferative vitreo-retinopathy (PVR)
Hemorrhage
Injury to relatively still healthy retina at the site of surgery

### 2. Product Safety:

Teratoma, tumor, and/or ectopic tissue development
Bulky graft of proliferating cells
Infection
Serious inflammatory immune reaction to the graft
Accelerated progression of GA
Progression to neovascular AMD in the study eye

### 3. Efficacy:

Duration of graft survival
Decreased rate of GA progression
Retinal sensitivity to light in engrafted regions and depth of scotomata
Visual acuity

## Claims

1. A pharmaceutical composition for use in a method of treating a subject with dry-form age-related macular degeneration (AMD), comprising:
administering into the subretina of the subject a therapeutically effective amount of the pharmaceutical composition comprising human retinal pigment epithelium (RPE) cells,
wherein at least 95% of the cells thereof co-express premelanosome protein (PMEL17) and cellular retinaldehyde binding protein (CRALBP), wherein the trans-epithelial electrical resistance of the cells in a monolayer is greater than 100 ohms to the subject, thereby treating the subject.

2. A pharmaceutical composition for use in a method of treating a subject with a retinal disease or condition, comprising:
administering into the retina of the subject a therapeutically effective amount of the pharmaceutical composition comprising human polygonal retinal pigment epithelium (RPE) cells,
wherein at least 95% of the cells thereof co-express premelanosome protein (PMEL17) and cellular retinaldehyde binding protein (CRALBP), wherein the trans-epithelial electrical resistance of the cells in a monolayer is greater than 100 ohms to the subject, wherein the therapeutically effective amount is between 50,000 - 5,000,000 cells per administration, thereby treating the subject.

3. A pharmaceutical composition for use in a method of treating a subject with a retinal disease or condition, comprising:
administering into the retina of the subject a therapeutically effective amount of a pharmaceutical composition comprising human retinal pigment epithelium (RPE cells) using a device, wherein the outer diameter of the device through which the cells are administered is between 90-100 µm, thereby treating the subject,
wherein at least 95% of the cells thereof co-express premelanosome protein (PMEL17) and cellular retinaldehyde binding protein (CRALBP), wherein the trans-epithelial electrical resistance of the cells in a monolayer is greater than 100 ohms to the subject.

4. The pharmaceutical composition of claim 1 for use in the method of claim 1, wherein the therapeutically effective amount is between 50,000-1,000,000 cells per administration.

5. The pharmaceutical composition of claim 2 for use in the method of claim 2, wherein the pharmaceutical composition comprises 500 cells per µl - 10,000 cells per µl.

6. The pharmaceutical composition of claim 2 for use in the method of claim 2, wherein said retinal disease or condition is selected from the group consisting of retinitis pigmentosa, retinal detachment, retinal dysplasia, retinal atrophy, retinopathy, macular dystrophy, cone dystrophy, cone-rod dystrophy, Malattia Leventinese, Doyne honeycomb dystrophy, Sorsby's dystrophy, pattern/butterfly dystrophies, Best vitelliform dystrophy, North Carolina dystrophy, central areolar choroidal dystrophy, angioid streaks, toxic maculopathy, Stargardt disease, pathologic myopia, retinitis pigmentosa, and macular degeneration.

7. The pharmaceutical composition of claim 6 for use in the method of claim 6, wherein said disease is age-related macular degeneration.

8. The pharmaceutical composition of claim 1 for use in the method of claim 1, wherein the subject fulfils at least one of the criteria selected from the group consisting of:
(i) is aged 55 or older;
(ii) has funduscopic findings of dry AMD with geographic atrophy in the macula, above 0.5 disc area in at least one eye;
(iii) is able to undergo a vitreoretinal surgical procedure under monitored anesthesia care; and
(iv) does not have an immunodeficiency disease.

9. The pharmaceutical composition of claim 1 for use in the method of claim 1, wherein the number of Oct4⁺TRA-1-60⁺ cells in the population is below 1:250,000.

10. The pharmaceutical composition of claim 1 for use in the method of claim 1, wherein at least 80% of the cells express Bestrophin 1, as measured by immunostaining and/or wherein at least 80 % of the cells express Microphthalmia-associated transcription factor (MITF), as measured by immunostaining and/or wherein more than 80 % of the cells express paired box gene 6 (PAX-6) as measured by FACS.

11. The pharmaceutical composition of claim 1 for use in the method of claim 1, wherein the cells secrete greater than 500 ng of Pigment epithelium-derived factor (PEDF) per ml per day and/or wherein the cells secrete PEDF and vascular endothelial growth factor (VEGF) in a polarized manner.

12. The pharmaceutical composition of claim 11 for use in the method of claim 11, wherein the ratio of apical secretion of PEDF: basal secretion of PEDF is greater than 1.

13. The pharmaceutical composition of claim 12 for use in the method of claim 12, wherein said ratio remains greater than 1 following incubation for 8 hours at 2-8°C.

14. The pharmaceutical composition of claim 1 for use in the method of claim 1 wherein said trans-epithelial electrical resistance of the cells remains greater than 100 ohms following incubation for 8 hours at 2-8°C.

15. The pharmaceutical composition of claim 11 for use in the method of claim 11, wherein the ratio of basal secretion of VEGF: apical secretion of VEGF is greater than 1.

16. The pharmaceutical composition of claim 1 for use in the method of claim 1, wherein the cells are capable of rescuing visual acuity in the RCS rat following subretinal administration.

17. The pharmaceutical composition of claim 1 for use in the method of claim 1, wherein the cells are generated by ex-vivo differentiation of human embryonic stem cells.

18. The pharmaceutical composition of claim 1 for use in the method of claim 1, wherein the cells are generated by:
(a) culturing human embryonic stem cells or induced pluripotent stem cells in a medium comprising nicotinamide so as to generate differentiating cells, wherein said medium is devoid of activin A;
(b) culturing said differentiating cells in a medium comprising nicotinamide and activin A to generate cells which are further differentiated towards the RPE lineage; and
(c) culturing said cells which are further differentiated towards the RPE lineage in a medium comprising nicotinamide, wherein said medium is devoid of activin A.

19. The pharmaceutical composition of claim 18 for use in the method of claim 18, wherein said embryonic stem cells or induced pluripotent stem cells are propagated in a medium comprising bFGF and TGFβ.

## Patentansprüche

1. Pharmazeutische Zusammensetzung zur Verwendung in einem Verfahren zur Behandlung eines Subjekts mit trockener Form der altersbedingten Makuladegeneration (age-related macular degeneration, AMD), umfassend:
Verabreichung einer therapeutisch wirksamen Menge der pharmazeutischen Zusammensetzung, die menschliche retinale Pigmentepithelzellen (RPE-Zellen) umfasst, in die Subretina des Patienten,
wobei mindestens 95 % der Zellen davon das Prämelanosomen-Protein (PMEL17) und das zelluläre Retinaldehyd-Bindungsprotein (CRALBP) coexprimieren,
wobei der transepitheliale elektrische Widerstand der Zellen in einer Monoschicht größer als 100 Ohm gegenüber dem Patienten ist, wodurch der Patient behandelt wird.

2. Pharmazeutische Zusammensetzung zur Verwendung in einem Verfahren zur Behandlung eines Subjekts mit einer Netzhauterkrankung oder einem Netzhautzustand, umfassend:
Verabreichung einer therapeutisch wirksamen Menge der pharmazeutischen Zusammensetzung, die menschliche polygonale retinale Pigmentepithelzellen (RPE) umfasst, in die Netzhaut des Patienten,
wobei mindestens 95% der Zellen davon das Prämelanosomen-Protein (PMEL17) und das zelluläre Retinaldehyd-Bindungsprotein (CRALBP) coexprimieren,
wobei der transepitheliale elektrische Widerstand der Zellen in einer Monoschicht größer als 100 Ohm gegenüber dem Subjekt ist, wobei die therapeutisch wirksame Menge zwischen 50.000 - 5.000.000 Zellen pro Verabreichung liegt, wodurch das Subjekt behandelt wird.

3. Pharmazeutische Zusammensetzung zur Verwendung in einem Verfahren zur Behandlung eines Subjekts mit einer Netzhauterkrankung oder einem Netzhautzustand, umfassend:
Verabreichung einer therapeutisch wirksamen Menge einer pharmazeutischen Zusammensetzung, die menschliches retinales Pigmentepithel (RPE-Zellen) umfasst, in die Netzhaut des Patienten unter Verwendung einer Vorrichtung, wobei der Außendurchmesser der Vorrichtung, durch die die Zellen verabreicht werden, zwischen 90-100 µm liegt, wodurch der Patient behandelt wird,
wobei mindestens 95 % der Zellen davon das Prämelanosomen-Protein (PMEL17) und das zelluläre Retinaldehyd-Bindungsprotein (CRALBP) coexprimieren,
wobei der transepitheliale elektrische Widerstand der Zellen in einer Monoschicht größer als 100 Ohm gegenüber dem Subjekt ist.

4. Pharmazeutische Zusammensetzung gemäß Anspruch 1 zur Verwendung in dem Verfahren gemäß Anspruch 1, wobei die therapeutisch wirksame Menge zwischen 50,000-1,000,000 Zellen pro Verabreichung liegt.

5. Pharmazeutische Zusammensetzung gemäß Anspruch 2 zur Verwendung bei dem Verfahren gemäß Anspruch 2, wobei die pharmazeutische Zusammensetzung 500 Zellen pro µl - 10,000 Zellen pro µl umfasst.

6. Pharmazeutische Zusammensetzung gemäß Anspruch 2 zur Verwendung bei dem Verfahren gemäß Anspruch 2, wobei die Erkrankung oder der Zustand der Netzhaut ausgewählt ist aus der Gruppe bestehend aus Retinitis pigmentosa, Netzhautablösung, Netzhautdysplasie, Netzhautatrophie, Retinopathie, Makuladystrophie, Zapfendystrophie, Zapfenstabdystrophie, Malattia Leventinese, Doyne-Waben-Dystrophie, Sorsby-Dystrophie, Muster/Schmetterlingsdystrophien, Best vitelliform Dystrophie, North Carolina Dystrophie, zentral areolare Choroidaldystrophie, Angioid Streaks, toxische Makulopathie, Stargardt Krankheit, pathologische Myopie, Retinitis pigmentosa und Makuladegeneration.

7. Pharmazeutische Zusammensetzung gemäß Anspruch 6 zur Verwendung in dem Verfahren gemäß Anspruch 6, wobei es sich bei der Krankheit um altersbedingte Makuladegeneration handelt.

8. Pharmazeutische Zusammensetzung gemäß Anspruch 1 zur Verwendung in dem Verfahren gemäß Anspruch 1, wobei das Subjekt mindestens eines der Kriterien erfüllt, ausgewählt aus der Gruppe bestehend aus:
(i) 55 Jahre oder älter ist;
(ii) einen funduskopischen Befund einer trockenen AMD mit geographischer Atrophie in der Makula aufweist, die in mindestens einem Auge über 0,5 des Scheibenbereichs (disc area) liegt;
(iii) in der Lage ist, sich einem vitreoretinalen chirurgischen Eingriff unter kontrollierter Anästhesiebehandlung zu unterziehen; und
(iv) nicht an einer Immunschwächekrankheit leidet.

9. Pharmazeutische Zusammensetzung gemäß Anspruch 1 zur Verwendung in dem Verfahren gemäß Anspruch 1, wobei die Anzahl der Oct4+TRA-1-60+-Zellen in der Bevölkerung unter 1:250,000 liegt.

10. Pharmazeutische Zusammensetzung gemäß Anspruch 1 zur Verwendung bei dem Verfahren gemäß Anspruch 1, wobei mindestens 80 % der Zellen Bestrophin 1, gemessen durch Immunfärbung, exprimieren und/oder wobei mindestens 80 % der Zellen den Mikrophthalmie-assoziierten Transkriptionsfaktor (MITF), gemessen durch Immunfärbung, exprimieren und/oder wobei mehr als 80 % der Zellen das Paired-Box-Gen 6 (PAX-6), gemessen durch FACS, exprimieren.

11. Pharmazeutische Zusammensetzung gemäß Anspruch 1 zur Verwendung in dem Verfahren gemäß Anspruch 1, wobei die Zellen mehr als 500 ng von Pigmentepithel-abgeleitetem Faktor (PEDF) pro ml pro Tag sezernieren und/oder wobei die Zellen PEDF und vaskulären endothelialen Wachstumsfaktor (VEGF) in einer polarisierten Weise sezernieren.

12. Pharmazeutische Zusammensetzung gemäß Anspruch 11 zur Verwendung in dem Verfahren gemäß Anspruch 11, wobei das Verhältnis der apikalen Sekretion von PEDF zu der basalen Sekretion von PEDF größer als 1 ist.

13. Pharmazeutische Zusammensetzung gemäß Anspruch 12 zur Verwendung in dem Verfahren gemäß Anspruch 12, wobei das Verhältnis nach 8-stündiger Inkubation bei 2-8°C größer als 1 bleibt.

14. Pharmazeutische Zusammensetzung gemäß Anspruch 1 zur Verwendung bei dem Verfahren gemäß Anspruch 1, wobei der transepitheliale elektrische Widerstand der Zellen nach 8-stündiger Inkubation bei 2-8°C größer als 100 Ohm bleibt.

15. Pharmazeutische Zusammensetzung gemäß Anspruch 11 zur Verwendung in dem Verfahren gemäß Anspruch 11, wobei das Verhältnis der basalen Sekretion von VEGF zu der apikalen Sekretion von VEGF größer als 1 ist.

16. Pharmazeutische Zusammensetzung gemäß Anspruch 1 zur Verwendung bei dem Verfahren gemäß Anspruch 1, wobei die Zellen in der Lage sind, die Sehschärfe bei der RCS-Ratte nach subretinaler Verabreichung zu retten.

17. Pharmazeutische Zusammensetzung gemäß Anspruch 1 zur Verwendung in dem Verfahren gemäß Anspruch 1, wobei die Zellen durch ex-vivo-Differenzierung von humanen embryonalen Stammzellen erzeugt werden.

18. Pharmazeutische Zusammensetzung gemäß Anspruch 1 zur Verwendung in dem Verfahren gemäß Anspruch 1, wobei die Zellen erzeugt werden durch:
(a) Kultivieren menschlicher embryonaler Stammzellen oder induzierter pluripotenter Stammzellen in einem Medium, das Nicotinamid enthält, um differenzierende Zellen zu erzeugen, wobei das Medium frei von Aktivin A ist;
(b) Kultivieren der differenzierenden Zellen in einem Medium, das Nicotinamid und Aktivin A enthält, um Zellen zu erzeugen, die weiter in Richtung der RPE-Linie differenziert werden; und
(c) Kultivieren der Zellen, die weiter in Richtung der RPE-Linie differenziert werden, in einem Medium, das Nicotinamid enthält, wobei das Medium frei von Aktivin A ist.

19. Pharmazeutische Zusammensetzung gemäß Anspruch 18 zur Verwendung in dem Verfahren gemäß Anspruch 18, wobei die embryonalen Stammzellen oder induzierten pluripotenten Stammzellen in einem Medium, das bFGF und TGFβ enthält, vermehrt werden.

## Revendications

1. Composition pharmaceutique pour utilisation dans un procédé de traitement d'un sujet atteint de dégénérescence maculaire liée à l'âge (DMLA) sèche, comprenant :
une administration dans la sous-rétine du sujet d'une quantité thérapeutiquement efficace de la composition pharmaceutique comprenant des cellules d'épithélium pigmentaire rétinien (EPR) humaines.
dans laquelle au moins 95 % des cellules de celle-ci co-expriment une protéine de prémélanosome (PMEL17) et une protéine de liaison de rétinaldéhyde cellulaire (CRALBP), dans laquelle la résistance électrique transépithéliale des cellules d'une monocouche est supérieure à 100 ohms pour le sujet, en traitant ainsi le sujet.

2. Composition pharmaceutique pour utilisation dans un procédé de traitement d'un sujet atteint d'une maladie ou d'une affection rétinienne, comprenant :
une administration dans la rétine du sujet d'une quantité thérapeutiquement efficace de la composition pharmaceutique comprenant des cellules d'épithélium pigmentaire rétinien (RPE) polygonales humaines,
dans laquelle au moins 95 % des cellules de celle-ci co-expriment une protéine de prémélanosome (PMEL17) et une protéine de liaison de rétinaldéhyde cellulaire (CRALBP), dans laquelle la résistance électrique transépithéliale des cellules d'une monocouche est supérieure à 100 ohms chez le sujet, dans laquelle la quantité thérapeutiquement efficace se situe entre 50 000 et 5 000 000 cellules par administration, en traitant ainsi le sujet.

3. Composition pharmaceutique pour utilisation dans un procédé de traitement d'un sujet atteint d'une maladie ou d'une affection rétinienne, comprenant :
une administration dans la rétine du sujet d'une quantité thérapeutiquement efficace de la composition pharmaceutique comprenant des cellules d'épithélium pigmentaire rétinien (RPE) humaines en utilisant un dispositif, dans laquelle le diamètre externe du dispositif à travers lequel les cellules sont administrées est entre 90 et 100 µm, en traitant ainsi le sujet,
dans laquelle au moins 95 % des cellules de celle-ci co-expriment une protéine de prémélanosome (PMEL17) et une protéine de liaison de rétinaldéhyde cellulaire (CRALBP), dans laquelle la résistance électrique transépithéliale des cellules d'une monocouche est supérieure à 100 ohms pour le sujet.

4. Composition pharmaceutique selon la revendication 1 pour utilisation dans le procédé de la revendication 1, dans laquelle la quantité thérapeutiquement efficace se situe entre 50 000 et 1 000 000 cellules par administration.

5. Composition pharmaceutique selon la revendication 2 pour utilisation dans le procédé de la revendication 2, dans laquelle la composition pharmaceutique comprend entre 500 cellules par µl et 10 000 cellules par µl.

6. Composition pharmaceutique selon la revendication 2 pour utilisation dans le procédé de la revendication 2, dans laquelle ladite maladie ou affection rétinienne est sélectionnée dans le groupe consistant en la rétinite pigmentaire, le détachement de la rétine, la dysplasie rétinienne, l'atrophie rétinienne, la rétinopathie, la dystrophie maculaire, la dystrophie des cônes, la dystrophie des cônes et des bâtonnets, la Malattia Leventinese, la dystrophie en nid d'abeille de Doyne, la dystrophie de Sorsby, la dystrophie maculaire en ailes de papillon, la dystrophie vitelliforme de Best, la dystrophie de Caroline du nord, la dystrophie centrale aréolaire de la choroïde, les stries angioïdes, la maculopathie toxique, la maladie de Stargardt, la myopie pathologique, la rétinite pigmentaire et la dégénérescence maculaire.

7. Composition pharmaceutique selon la revendication 6 pour utilisation dans le procédé de la revendication 6, dans laquelle ladite maladie est la dégénérescence maculaire liée à l'âge.

8. Composition pharmaceutique selon la revendication 1 pour utilisation dans le procédé de la revendication 1, dans laquelle le sujet remplit au moins l'un des critères sélectionnés dans le groupe consistant en :
(i) le sujet est âgé de 55 ans ou plus
(ii) le sujet a des résultats de fondoscopie diagnostiquant la DMLA sèche avec atrophie géographique dans la macula, à 0,5 au-dessus de la zone de disque dans au moins un œil ;
(iii) le sujet est en mesure de subir une intervention chirurgicale vitréo-rétinienne sous anesthésie surveillée ; et
(iv) le sujet n'est pas atteint d'une maladie immunodéficience.

9. Composition pharmaceutique selon la revendication 1 pour utilisation dans le procédé de la revendication 1, dans laquelle le nombre de cellules Oct4⁺TRA-1-60⁺ dans la population est inférieur à 1 :250 000.

10. Composition pharmaceutique selon la revendication 1 pour utilisation dans le procédé de la revendication 1, dans laquelle au moins 80 % des cellules expriment la Bestrophine 1, telle que mesurée par immunomarquage et/ou dans laquelle au moins 80 % des cellules expriment le facteur de transcription associé à la microphtalmie (MITF), tel que mesuré par immunomarquage et/ou dans laquelle plus de 80% des cellules expriment le gène à boîte *paired* 6 (PAX-6) tel que mesuré par CMF.

11. Composition pharmaceutique selon la revendication 1 pour utilisation dans le procédé de la revendication 1, dans laquelle les cellules secrètent plus de 500 ng de facteur dérivé d'épithélium pigmentaire (PEDF) par ml par jour et/ou dans laquelle les cellules secrètent le PEDF et le facteur de croissance d'endothélium vasculaire (VEGF) de manière polarisée.

12. Composition pharmaceutique selon la revendication 11 pour utilisation dans le procédé la revendication 11, dans laquelle le rapport sécrétion apicale de PEDF : sécrétion basale de PEDF est supérieur à 1.

13. Composition pharmaceutique selon la revendication 12 pour utilisation dans le procédé de la revendication 12, dans laquelle le rapport reste supérieur à 1 après incubation pendant 8 heures entre 2 et 8°C.

14. Composition pharmaceutique selon la revendication 1 pour utilisation dans le procédé de la revendication 1, dans laquelle ladite résistante électrique transépithéliale des cellules reste supérieure à 100 ohms après incubation pendant 8 heures entre 2 et 8°C.

15. Composition pharmaceutique selon la revendication 11 pour utilisation dans le procédé de la revendication 11, dans laquelle le rapport sécrétion basale de VEGF : sécrétion apicale de VEGF est supérieur à 1.

16. Composition pharmaceutique selon la revendication 1 pour utilisation dans le procédé de la revendication 1, dans laquelle les cellules sont capables de rétablir une acuité visuelle chez le rat RCS après administration sous-rétinienne.

17. Composition pharmaceutique selon la revendication 1 pour utilisation dans le procédé de la revendication 1, dans laquelle les cellules sont générées par différenciation ex-vivo de cellules souches embryonnaires humaines.

18. Composition pharmaceutique selon la revendication 1 pour utilisation dans le procédé de la revendication 1, dans laquelle les cellules sont générées par :
(a) culture de cellules souches embryonnaires humaines ou de cellules souches pluripotentes induites dans un milieu comprenant du nicotinamide afin de générer des cellules différentiées, dans laquelle ledit milieu est dépourvu d'activine A ;
(b) culture desdites cellules différentiées dans un milieu comprenant du nicotinamide et de l'activine A pour générer des cellules qui sont différentiées davantage vers la lignée RPE,
(c) culture desdites cellules qui sont différentiées davantage vers la lignée RPE dans un milieu comprenant du nicotinamide, dans laquelle ledit milieu est dépourvu d'activine A.

19. Composition pharmaceutique selon la revendication 18 pour utilisation dans le procédé de la revendication 18, dans laquelle lesdites cellules souches embryonnaires ou lesdites cellules souches pluripotentes induites sont propagées dans un milieu comprenant bFGF et TGFβ.
